# FASCICULE DE BREVET EUROPEEN

(11) **EP 1 556 414 B1**
(45) Date de publication et mention de la délivrance du brevet: **07.03.2012**
(21) Numéro de dépôt: 03807882.0
(22) Date de dépôt: 08.10.2003
(51) Int. Cl.: C08B 37/10, A61K 31/727

(54) **MELANGES D'OLIGOSACCHARIDES DERIVES D'HEPARINE, LEUR PREPARATION ET COMPOSITIONS PHARMACEUTIQUES LES CONTENANT**
MISCHUNGEN VON AUS HEPARIN HERGESTELLTEN OLIGOSACCHARIDEN, DEREN HERSTELLUNG UND DIESE ENTHALTENDEN PHARMAZEUTISCHEN ZUSAMMENSETZUNGEN
HEPARIN-DERIVED OLIGOSACCHARIDE MIXTURES, PREPARATION THEREOF AND PHARMACEUTICAL COMPOSITIONS CONTAINING THE SAME

(30) Priorité: 10.10.2002 FR 0212584
(43) Date de publication de la demande: 27.07.2005
(73) Titulaire: Aventis Pharma S.A., 92160 Antony (FR)
(72) Inventeur: BIBEROVIC, Vesna, F-93600 AULNAIS-SOUS-BOIS (FR); GRONDARD, Luc, F-91080 COURCOURONNES (FR); MOURIER, Pierre, F-94220 CHARENTON LE PONT (FR); VISKOV, Christian, F-91130 RIS ORANGIS (FR)
(74) Mandataire: Romanowski, Caroline
(86) Numéro de dépôt international: PCT/FR2003/002960
(87) Numéro de publication internationale: WO 2004/033503

(56) Documents cités:
- EP-A- 0 027 089
- WO-A-02/08295
- US-B1- 6 384 021

## Description

La présente invention concerne des mélanges d'oligosaccharides dérivés d'héparine, leur procédé de préparation et les compositions pharmaceutiques les contenant.

L'héparine est un mélange de mucopolysaccharides sulfatés d'origine animale, utilisée notamment pour ses propriétés anticoagulantes et antithrombotiques.

L'héparine présente cependant des inconvénients qui limitent les conditions de son utilisation. En particulier, son activité anticoagulante importante (anti IIa) peut occasionner des hémorragies.

Des héparines de bas poids moléculaire obtenues par dépolymérisation basique d'esters d'héparine ont été proposées (EP40144) ; cependant ces produits présentent encore une activité anti-IIa importante.

Des héparines de très bas poids moléculaire ont également été décrites dans US6384021. Cependant les valeurs d'activité anti-Xa obtenues dans les exemples décrits ne dépassent pas les 120 UI, et le ratio anti-Xa/anti-IIa obtenu est compris entre 15 et 50.

Dans WO-0208295, des héparines de très bas poids moléculaires sont préparés par un procédé différent de US6384021 et présentent une activité comprise entre 100 et 150 UI avec des ratio anti-Xa/anti-IIa également très élevés pour certains exemples d'application.

La demande de Brevet Européen EP-A-027089 divulgue des fractions d'oligosaccharides possédant une activité anti-Xa supérieure à celle de l'héparine non fractionnée.

Il existe toutefois un besoin constant dans cette classe de médicament d'améliorer les activités anti-Xa, notamment en obtenant des activités supérieures à 150 UI/mg, ainsi que le ratio anti-Xa/anti-IIa, et donc de mettre au point de nouvelles générations de dérivés d'Héparines.

Un des objectifs de l'invention est donc d'améliorer l'activité anti-Xa et le ratio anti-Xa/anti-IIa en modifiant les procédés décrits dans l'art antérieur, notamment en contrôlant le pourcentage d'eau lors de l'étape de dépolymérisation. Les héparines ainsi obtenues présentent ainsi une excellente activité antithrombotique et possèdent une activité aXa voisine de celle de l'héparine tout en diminuant les risques d'hémorragie avec une très faible activité aIIa. De même, les produits de l'invention présentent des durées de demi-vie très supérieures à celle de l'héparine.

L'invention a donc pour objet de nouveaux mélanges d'oligosaccharides dérivés d'héparine possédant une activité plus sélective vis-à-vis du facteur X activé (facteur Xa) et du facteur II activé (facteur IIa) que l'héparine.

Il est entendu que les mélanges de polysaccharides ayant un poids moléculaire moyen de 1500 à 3000 Da peuvent être qualifiés d'oligosaccharides.

La présente invention a donc pour objet les mélanges d'oligosaccharides sulfatés possédant la structure générale des polysaccharides constitutifs de l'héparine et présentant les caractéristiques suivantes :
- ils ont un poids moléculaire moyen de 2000 à 3000 daltons, une activité anti-Xa > 150 UI/mg et < 200 UI/mg, une activité anti-IIa inférieure à 10 UI/mg et un rapport activité anti-Xa/activité anti-IIa supérieur à 30,
- les oligosaccharides constitutifs des mélanges contiennent 2 à 26 motifs saccharidiques, ont un motif acide uronique-4,5 insaturé 2-O-sulfate à l'une de leurs extrémités, contiennent une fraction hexasaccharide représentant de 15 à 25 % des mélanges d'oligosaccharides, et contiennent de 8 à 15 % de l'hexasaccharide ΔIIa-IIs-Is dans la fraction hexasaccharide :

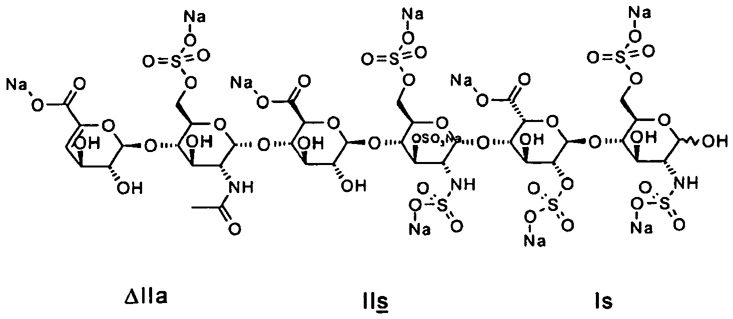

L'hexasaccharide ΔIIa-IIs-Is contenu dans le mélange d'oligosaccharides décrit dans la présente invention est une séquence fortement affine à l'ATIII et caractérisé par une activité aXa supérieure à 740 U/mg.

Le mélange d'oligosaccharides décrit dans la présente invention est sous forme d'un sel de métal alcalin ou alcalinoterreux.

Comme sel de métal alcalin ou alcalinoterreux, sont préférés les sels de sodium, potassium, calcium et magnésium.

Le poids moléculaire moyen est déterminé par chromatographie liquide haute pression en utilisant deux colonnes en série par exemple celles commercialisées sous le nom de TSK G3000 XL et TSK G2000 XL. La détection est réalisée par réfractométrie. L'éluant utilisé est du nitrate de lithium et le débit est de 0,6 ml/min. Le système est calibré avec des standards préparés par fractionnement de l'Enoxaparine (AVENTIS) par chromatographie sur gel d'agarose-polyacrylamide (IBF). Cette préparation est réalisée selon la technique décrite par Barrowcliffe et al, Thromb. Res., 12, 27-36 (1977-78) ou D.A. Lane et al, Thromb. Res., 12, 257-271 (1977-78). Les résultats sont calculés à l'aide du logiciel GPC6 (Perkin Elmer).

L'activité anti-Xa est mesurée par la méthode amydolytique sur un substrat chromogénique décrite par Teien et al, Thromb. Res., 10, 399-410 (1977), avec comme étalon le premier étalon international des héparines de bas poids moléculaire.

L'activité anti-IIa est mesurée par la technique décrite par Anderson L.O. et al, Thromb. Res., 15, 531-541 (1979), avec comme étalon le premier étalon international des héparines de bas poids moléculaire.

La fraction hexasaccharide représente de 15 à 25 % du mélange d'oligosaccharides.

Les mélanges selon l'invention contiennent de 8 à 15 % de l'hexasaccharide ΔIIa-IIs-Is dans la fraction hexasaccharide du mélange d'oligosaccharides.

Le pourcentage de la fraction hexasaccharide peut être déterminée de façon analytique par chromatographie liquide haute pression sur colonnes TSK G3000 XL et TSK G2000 XL ou bien par séparation préparative de la fraction hexasaccharide. Le mélange est dans ce cas chromatographié sur des colonnes remplies de gel de type polyacrylamide agarose tel que celui commercialisé sous la marque Ultrogel ACA202^{R} (Biosepra). Le mélange est élué par une solution d'hydrogénocarbonate de sodium. De préférence, la solution d'hydrogénocarbonate de sodium est une solution de 0,1 mol/l à 1 mol/l. Encore plus préférentiellement, la séparation est réalisée a une concentration de 1 mole/l. La détection est réalisée par spectrométrie UV (254nm). Après fractionnement, la fraction hexasaccharide en solution dans l'hydrogénocarbonate de sodium est neutralisée par de l'acide acétique glacial. La solution est ensuite concentrée sous pression réduite de façon à obtenir une concentration en acétate de sodium supérieure à 30 % en poids. La fraction d'hexasaccharide est précipitée par addition de 3 à 5 volumes de méthanol. La fraction d'hexasaccharides est récupérée par filtration sur verre fritté n°3. Le mélange d'hexasaccharides obtenu peut être analysé par CLHP (Chromatographie Liquide Haute Performance) pour en déterminer la teneur en hexasaccharide ΔIIa-IIs-Is. L'hexasaccharide ΔIIa-IIs-Is peut être isolé par chromatographie CLHP préparative ou par chromatographie d'affinité sur colonne antithrombine III sepharose selon les techniques utilisées par l'homme du métier (M. Hook, I. Bjork, J. Hopwood and U. Lindahl, F.E B.S letters, vol 656(1) (1976)).

De préférence, les mélanges selon l'invention ont une activité anti-IIa inférieure à 5 UI/mg, et tout particulièrement de 0,5 à 3,5 UI/mg. Les exemples d'applications décrits plus bas mettent en évidence des valeurs comprises entre 1,1 et 1,6 UI/mg lorsqu'on met en oeuvre les caractéristiques préférées du procédé.

De préférence, les mélanges présentent un rapport activité anti-Xa/activité anti-IIa supérieur à 50 et tout particulièrement supérieur à 100.

De préférence, les mélanges selon l'invention ont un poids moléculaire moyen compris entre entre 2400 et 2650 Da.

L'invention a donc tout particulièrement pour objet les mélanges tels que définis précédemment présentant une activité anti-Xa > 150 UI/mg et < 200 UI/mg, une activité anti-IIa comprise entre 0,5 et 3,5 UI/mg et un poids moléculaire moyen compris entre 2400 et 2650 Da.

Les mélanges d'oligosaccharides selon l'invention peuvent être préparés par dépolymérisation d'un sel d'ammonium quaternaire de l'ester benzylique de l'héparine en milieu organique, au moyen d'une base organique forte de pKa supérieur à 20 (propriétés de préférence apparentées à la famille des phosphazènes définies par exemple selon R. Schwesinger et al, Angew. Chem. Int. Ed. Engl. 26, 1167-1169 (1987), R. Schwesinger et al, Angew. Chem. 105, 1420 (1993)), transformation du sel d'ammonium quaternaire de l'ester benzylique de l'héparine dépolymérisée en sel de sodium, saponification des esters résiduels et éventuellement purification. Le procédé selon l'invention reprend les grandes étapes du procédé tel que décrit dans W0 0208295 tout en ajoutant une caractéristique essentielle qui permet d'obtenir les mélanges d'oligosaccharides selon l'invention avec les caractéristiques physico-chimiques et les activités décrites plus haut.

En effet, pour obtenir les mélanges d'oligosaccharides spécifique selon l'invention, il est nécessaire de contrôler la sélectivité de la base par un contrôle très précis de la teneur en eau du mélange pendant l'étape de dépolymérisation.

Le procédé selon la présente invention est en effet caractérisé par un contrôle de la forte sélectivité de la base lors de la dépolymérisation. Elle permet de dépolymériser l'héparine tout en préservant au maximum les séquences affines à l'ATIII tel que l'hexasaccharide ΔIIa-IIs-Is décrit dans la présente invention. Cette étape critique du procédé permet d'obtenir les mélanges d'oligosaccharides selon l'invention.

Cette caractéristique du procédé se traduit par des activités aXa inattendues au regard du poids moléculaire moyens des mélanges d'oligosaccharides (150 UI/mg < aXa < 200 UI/mg pour un poids moléculaire moyen compris entre 2000 Da et 3000 Da). Cette sélectivité est due aux caractéristiques physico-chimiques très particulières des bases phosphazène qui présentent un pKa supérieur à 20, un très fort encombrement stérique et une faible nucléophilicité.

Cet effet s'exprime pleinement lorsque le milieu réactionnel est anhydre. Par contre, lorsque la teneur en eau du milieu réactionnel augmente, on observe une diminution drastique de la sélectivité de la dépolymérisation. La préservation des séquences affines à l'ATIII diminue et la conséquence est une chute importante de l'activité aXa. En présence de faible quantité d'eau, la base phosphazène se protone et l'espèce réactive devient un hydroxyde d'ammonium quaternaire. Dans ce cas, les propriétés de très fort encombrement stérique et faible nucléophilicité sont perdues et influent fortement sur la qualité du produit obtenu. Lorsque l'on réalise des essais de dépolymérisation avec une teneur en eau mesurée et contrôlée, on peut voir cet effet pleinement s'exprimer.

Le tableau suivant résume l'impact de la teneur en eau sur la sélectivité de la dépolymérisation (seul ce paramètre est variable dans les essais : les stoechiométries des réactifs, les dilutions, les températures restent constantes selon les critères de l'homme de l'art. La base utilisée est la base phosphazène : 2-tert-butylimino-2-diéthylamino-1,3 diméthylperhydro-1,2,3-diaza-phosphorine).

| Teneur en eau % | 0,05% | 0,1% | 0,2% | 0,3% | 0,4% | 0,57% | 1,8% | 2,5% |
|---|---|---|---|---|---|---|---|---|
| aXa UI/mg | 192 | 177 | 161 | 132 | 122 | 120 | 105 | 99 |
| aIIa UI/mg | 1,3 | 1,5 | 1,4 | 1,4 | 1,3 | 1,4 | 3,1 | 13,4 |
| aXa/aIIa | 148 | 118 | 115 | 94 | 94 | 85,7 | 34 | 7,4 |

Pour une sélectivité optimale et une préservation maximale des séquences affines à l'ATIII, il est préférable d'opérer à des teneurs en eau inférieures à 0,3 % lorsque l'on travaille avec 1 équivalent molaire de base phosphazène par rapport à l'ester benzylique de l'héparine, sel de benzéthonium.

L'invention a donc tout particulièrement pour objet l'étape de dépolymérisation du sel d'ammonium quaternaire de l'ester benzylique de l'héparine obtenue selon les méthodes connues de l'homme du métier caractérisé en ce qu'on utilise une base de la famille des phosphazènes, notamment en solution dichlorométhane, renfermant un pourcentage d'eau inférieur à 0,3 % et tout particulièrement inférieur à 0,2 %.

Avantageusement, le rapport en mole base forte/ester est compris entre 0,2 et 5, de préférence entre 0,6 et 2 et tout particulièrement entre 0,8 et 1,2. L'utilisation du rapport équimoléculaire fait donc partie des modes d'exécution préférés de l'invention.

D'autres solvants aprotiques connus de l'homme du métier peuvent être utilisés tels que le THF ou le DMF.

Le sel d'ammonium quaternaire de l'ester benzylique de l'héparine est de préférence le sel de benzéthonium, de cétylpyridinium ou de cétyltriméthyl ammonium.

Les bases de la famille des phosphazènes sont, de préférence, celles de formule : 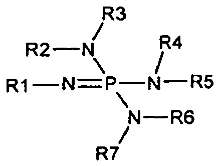 dans laquelle les radicaux R1 à R7 sont identiques ou différents et représentent des radicaux alkyle.

En particulier l'invention a pour objet le procédé tel que défini plus haut caractérisé en ce que la base utilisée dans l'étape d) de dépolymérisation) est le 2-tert-butylimino-2-diethylamino-1,3-diméthylperhydro-1,2,3-diaza-phosphorine (1,3,2-diazaphosphorin-2-amine, 2-[(1,1-dimethylethyl)imino]-N,N-diéthyl-1,2,2,2,3,5,6-octahydro-1,3-diméthyl) : 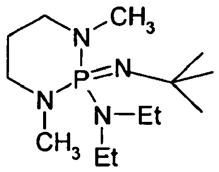 ou le tert-butylimino-tri(pyrrolidino)-phosphorane.

Dans les formules précédentes, les radicaux alkyle contiennent 1 à 6 atomes de carbone en chaîne droite ou ramifiée.

L'invention a donc pour objet un procédé de préparation des mélanges d'oligosaccharides selon l'invention comprenant les étapes suivantes :
a) Transalification de l'héparine de sodium par action de chlorure de benzéthonium,
b) Estérification de l'héparinate de benzéthonium par action de chlorure de benzyle,
c) Transalification de l'ester benzylique obtenu en sel d'ammonium quaternaire,
d) Dépolymérisation du sel d'ammonium quaternaire de l'ester benzylique de l'héparine par la méthode telle que définie plus haut,
e) Transformation du sel d'ammonium quaternaire en sel de sodium,
f) Eventuellement saponification de l'héparine par action d'une base telle que la soude
g) Eventuellement purification notamment par action d'un agent d'oxydation tel que l'eau oxygénée.

Le schéma réactionnel suivant illustre la présente invention :

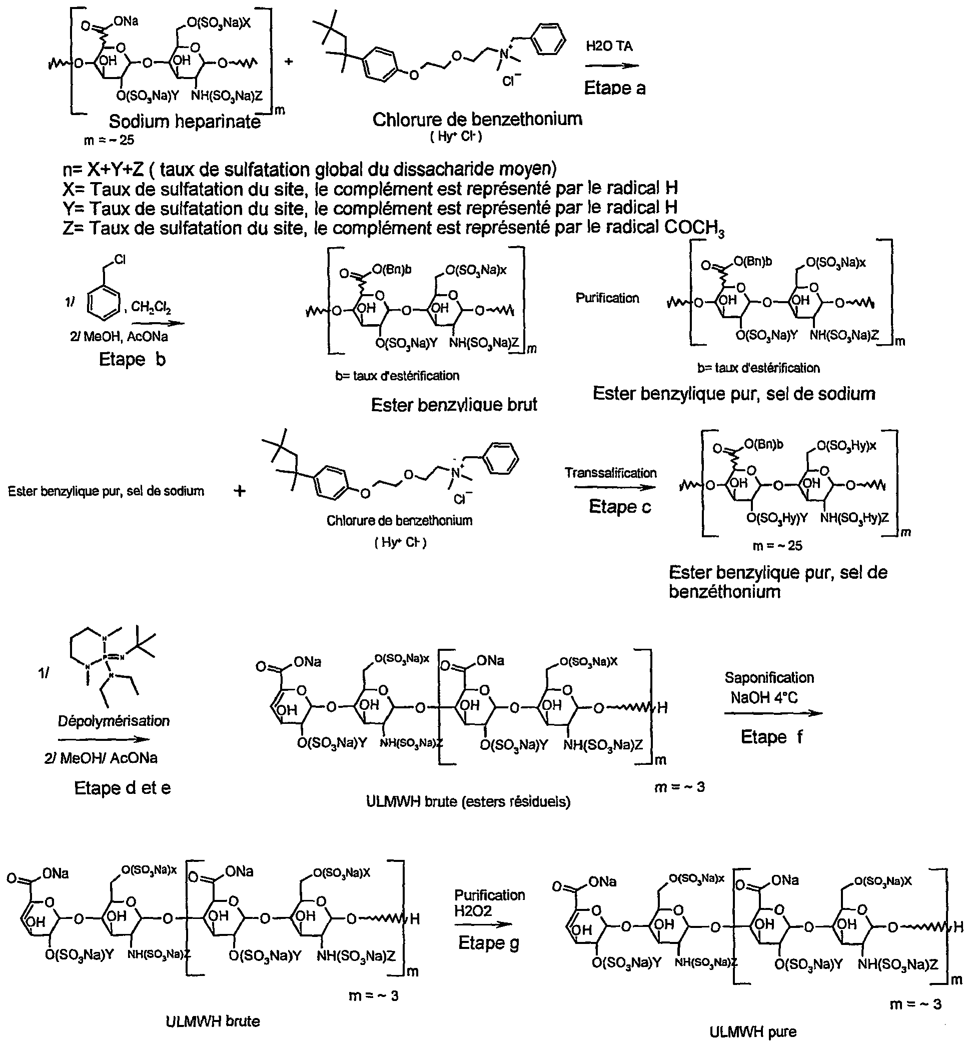

La transformation du sel d'ammonium quaternaire de l'ester benzylique de l'héparine dépolymérisée en sel de sodium (étape e) est effectuée, généralement, par traitement du milieu réactionnel avec une solution alcoolique d'acétate de sodium et, de préférence avec une solution à 10 % d'acétate de sodium dans le méthanol (poids/volume), à une température comprise entre 15 et 25°C. L'équivalent en poids d'acétate ajouté est préférentiellement 3 fois supérieur à la masse de sel d'ammonium quaternaire de l'ester benzylique de l'héparine engagé dans la réaction de dépolymérisation.

La saponification (étape f) s'effectue généralement au moyen d'un hydroxyde de métal alcalin tel que la soude, la potasse, l'hydroxyde de lithium, en milieu aqueux, à une température comprise entre 0 et 20°C et de préférence 0 et 10°C. On utilisera de façon générale de 1 à 5 équivalents molaires d'hydroxyde de métal alcalin. De préférence, la saponification sera réalisée en présence de 1 à 2 équivalents molaires d'hydroxyde de métal alcalin.

Le produit final peut éventuellement être purifié (étape g) par toute méthode connue de purification des héparines dépolymérisées (par exemple EP 0037319B1). De préférence, on purifie au moyen de peroxyde d'hydrogène, en milieu aqueux, à une température de 10 à 50°C. De préférence, cette opération sera réalisée entre 20 et 40°C.

Le sel d'ammonium quaternaire de l'ester benzylique de l'héparine peut être préparé selon le schéma réactionnel suivant :
a) transformation de l'héparine de sodium au moyen du chlorure de benzéthonium pour obtenir l'héparinate de benzéthonium (Transalification),
b) estérification du sel de benzéthonium obtenu précédemment au moyen du chlorure de benzyle puis traitement par une solution alcoolique d'acétate de sodium pour obtenir le sel de sodium de l'ester benzylique de l'héparine,
c) transalification du sel de sodium de l'ester benzylique de l'héparine en sel d'ammonium quaternaire et, de préférence en sel de benzéthonium, de cétyl pyrinium ou de cétyltriméthyl ammonium.

La réaction de l'étape a) est réalisée par action du chlorure de benzéthonium en excès, sur l'héparine sodique, à une température voisine de 15 à 25°C. De manière avantageuse, le rapport molaire sel/héparine sodique est compris entre 3 et 4.

L'héparine de départ utilisée est de préférence une héparine de porc. Celle-ci peut être préalablement purifiée pour diminuer son taux de sulfate de dermatan selon le procédé décrit dans le brevet FR2663639.

L'estérification de l'étape b) s'effectue de préférence au sein d'un solvant organique chloré (chloroforme, chlorure de méthylène par exemple), à une température comprise entre 25 et 45°C et, de préférence entre 30 et 40°C. L'ester sous forme de sel de benzéthonium est ensuite récupéré sous forme de sel de sodium par précipitation au moyen d'acétate de sodium à 10 % en poids dans un alcool tel que le méthanol. On emploie généralement 1 à 1,2 volume d'alcool par volume de milieu réactionnel. La quantité de chlorure de benzyle et le temps de réaction sont adaptés pour obtenir un taux d'estérification compris entre 50 et 100 % et, de préférence, entre 70 et 90 %. De façon préférentielle, on utilise 0,5 à 1,5 parties en poids du chlorure de benzyle pour 1 partie en poids du sel de benzéthonium de l'héparine. De même, de façon préférentielle, le temps de réaction sera compris entre 10 et 35 h.

La transalification de l'étape c) s'effectue au moyen d'un chlorure d'ammonium quaternaire et, de préférence, au moyen de chlorure de benzéthonium, de chlorure de cétylpyridinium ou de chlorure de cétyltriméthyl ammonium, en milieu aqueux, à une température comprise entre 10 et 25°C. De manière avantageuse, le rapport en mole chlorure d'ammonium quaternaire/sel de sodium de l'ester benzylique de l'héparine est compris entre 2 et 3.

Les mélanges selon l'invention sous forme de sel de sodium, peuvent être transformés en un autre sel d'un métal alcalin ou alcalinoterreux. On passe éventuellement d'un sel à l'autre en utilisant la méthode décrite dans le brevet FR 73 13 580.

Les mélanges selon l'invention ne sont pas toxiques et peuvent ainsi être utilisés comme médicaments.

Les mélanges d'oligosaccharides de la présente invention peuvent être utilisés comme agents antithrombotiques. En particulier, ils sont utiles pour le traitement ou la prévention des thromboses veineuses et artérielles, la thrombose veineuse profonde, l'embolie pulmonaire, l'angor instable, l'infarctus du myocarde, l'ischémie cardiaque, les maladies occlusives des artères périphériques et la fibrillation auriculaire. Ils sont également utiles dans la prévention et le traitement de la prolifération des cellules musculaires lisses, l'athérosclérose et l'artériosclérose, pour le traitement et la prévention du cancer par la modulation de l'angiogénèse et des facteurs de croissance, et pour le traitement et la prévention des désordres diabétiques tel que les rétinopathies et néphropathies diabétiques.

La présente invention concerne également les compositions pharmaceutiques contenant comme principe actif un mélange de formule (I) éventuellement en association avec un ou plusieurs excipients inertes.

Les compositions pharmaceutiques sont par exemple des solutions injectables par voie sous-cutanée ou intraveineuse. Elles sont également utiles pour une administration par voie pulmonaire (inhalation) ou par voie orale.

La posologie peut varier en fonction de l'âge, du poids et de l'état de santé du patient. Pour un adulte, elle est en général comprise entre 20 et 100 mg par jour par voie intramusculaire ou sous-cutanée.

Les exemples suivants illustrent l'invention sans toutefois la limiter.

### Exemple A : PREPARATION D'HEPARINATE DE BENZYLE SEL DE BENZETHONIUM

### Héparinate de benzéthonium

A une solution de 10 g d'héparine de sodium dans 100 ml d'eau, on ajoute une solution de 25 g de chlorure de benzéthonium dans 125 ml d'eau. Le produit est filtré, lavé à l'eau et séché.

### Ester benzylique d'héparine (sel de sodium)

A une solution de 20 g d'héparinate de benzéthonium dans 80 ml de chlorure de méthylène, on ajoute 16 ml de chlorure de benzyle. La solution est chauffée à une température de 30°C pendant 12 heures. On ajoute alors 108 ml d'une solution à 10 % d'acétate de sodium dans le méthanol, filtre, lave au méthanol et sèche. On obtient ainsi 7,6 g d'ester benzylique d'héparine sous forme de sel de sodium dont le taux d'estérification est de 77 %.

### Ester benzylique de l'héparine (sel de benzéthonium)

Dans un erlenmeyer A de 2 litres, on introduit 36 g (0,0549 mole) d'ester benzylique d'héparine (sel de sodium) et 540 ml d'eau distillée. Après homogénéisation à une température d'environ 20°C, on obtient une solution jaune pâle. Sous agitation magnétique, on prépare dans un erlenmeyer B de 1 litre, une solution de 64,45 g (0,1438 mole) de chlorure de benzéthonium et de 450 ml d'eau. La solution de l'erlenmeyer B est coulée en environ 35 minutes dans la solution de l'erlenmeyer A sous agitation. On observe la formation d'un précipité blanc abondant. L'erlenmeyer B est rincé avec 200 ml d'eau distillée et l'eau de lavage est introduite dans l'erlenmeyer A. L'agitation est alors arrêtée et on laisse déposer la suspension pendant 12 heures. Ce temps écoulé, on prélève et on écarte la partie limpide du surnageant. Sur le précipité sédimenté (aspect de bouillie), on ajoute 560 ml d'eau et on agite pendant 20 minutes. On laisse re-sédimenter le précipité en 30 minutes environ. Le surnageant est prélevé et écarté (560 ml). Sur le précipité sédimenté, on renouvelle deux fois cette opération de lavage par environ 560 ml d'eau distillée. Dans la dernière opération de lavage, on laisse le précipité en suspension et on filtre sur Verre fritté 3. Le gâteau est ensuite lavé par 4 fois 200 ml d'eau distillée. Le solide blanc humide est essoré puis séché sous pression réduite (2,7 kPa), à une température voisine de 60°C. Après 12 heures de séchage, on obtient 87,5 g d'héparinate de benzyle, sel de benzéthonium. Le rendement obtenu est de 94,9 %.

### Exemple B : DESCRIPTION DE l'HEXASACCHARIDE ATIII (ΔIIa-IIs-Is)

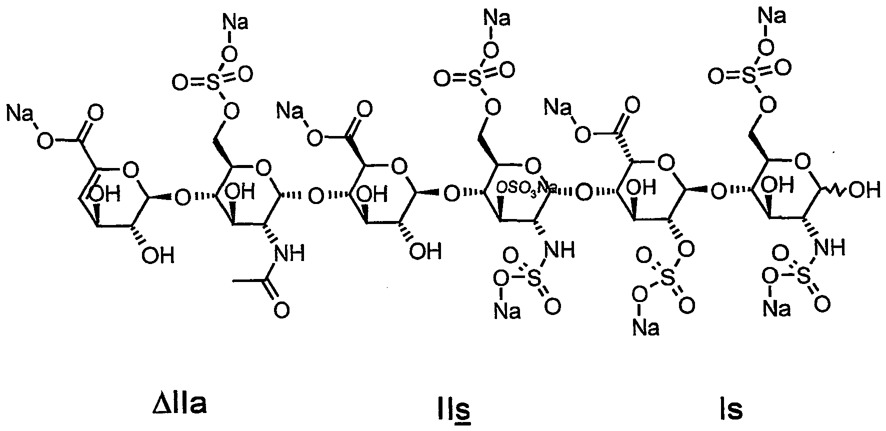

Spectre proton dans D₂O, 500 MHz, T=298 K, δ en ppm : 1,97 (3H, s), 3,18 (1H, dd, 10 et 3Hz), 3,30 (1H, t, 8Hz), 3,37 (1H, dd, 10 et 3Hz), 3,60 (2H, m), entre 3,65 et 3,85 (6H, m), 3,87 (2H, m), 3,95 (1H, d, 8Hz), 4,03 (1H, d, 8Hz), entre 4,05 et 4,13 (4H, m), entre 4,16 et 4,45 (8H, m), 4,52 (1H, d, 8Hz), 4,67 (1H, m), 5,06 (1H, d, 6Hz), 5, 10 (1H, d, 3Hz), 5,33 (1H, d, 4Hz), 5,36 (1H, d, 3Hz), 5,46 (1H, d, 3Hz), 5,72 (1H, d, 4Hz).

Sel de décasodium de l'acide 4-désoxy -α-L-thréo-hexenepyranosyluronique-(1→ 4)-2-désoxy-2-acétamido-6-O-sulfo-α-D-glucopyranosyl-(1→4)-acide P-D-glucopyranosyluronique-(1→4)-2-désoxy-2-sulfamido-3,6-di-O-sulfo-α-D-glucopyranosyl)-(1→4)-acide 2-O-sulfo-α-Z-idopyrano-syluronique-(1→4)-2-desoxy-2-sulfamido-6-O-sulfo-α-D-glucopyranose.
- Les exemples 1 à 7 et 12 illustrent l'influence de la teneur en eau sur la sélectivité de la réaction de polymérisation et l'activité aXa et aIIa des produits obtenus.
- Les exemples 8 à 10 illustrent l'influence du nombre d'équivalents de base sur l'activité aXa et aIIa du produit obtenu (avec une teneur en eau de 0,1%)

- L'exemple 11 illustre l'utilisation d'un autre base phosphazene que le 2-tert-butylimino-2-diethylamino-1,3-diméthylperhydro-1,2,3-diaza-phosphorine :
   Utilisation du tert-butylimino tri(pyrrolidino) phosphorane

### EXEMPLE 1

### Dépolymérisation et transformation en sel de sodium ( 0,1 % d'eau ) :

Dans un erlenmeyer A, on introduit 70 ml de dichlorométhane. Sous agitation, on charge lentement 10 g (0,006 mole) d'ester benzylique d'héparine (taux d'estérification : 75 %, sel de benzéthonium) obtenu comme décrit dans l'exemple A. La teneur en eau du milieu réactionnel est ajustée 0,1 %. La solution est chauffée à 40°C sous azote. Après dissolution complète, on revient à une température voisine de 20°C puis on ajoute 1,75 ml (0,006 mole) de 2-tert-butylimino-2-diethylamino-1,3-diméthylperhydro-1,2,3-diaza-phosphorine. On agite à une température voisine de 20°C, pendant 24 heures. Pendant ce temps, on prépare dans un erlenmeyer B, une solution de 30 g d'acétate de sodium anhydre dans 300 ml de méthanol. Après dissolution complète, on ajoute à la solution 5 g de célite Hyflo supercel. Sous agitation magnétique, le mélange réactionnel de l'erlenmeyer A est coulé en 1 minute 30 secondes dans la solution méthanolique d'acétate de sodium à une température voisine de 5°C. Après 5 minutes d'agitation on laisse décanter la suspension pendant une heure 30 minutes. La partie limpide du surnageant est prélevée et écartée (220 ml). Sur le précipité sédimenté, on ajoute 220 ml de méthanol et on agite pendant 5 minutes. On laisse re-sédimenter le précipité 1 heure 20 minutes environ. Le surnageant est prélevé et écarté (250 ml). Sur le précipité sédimenté, on ajoute 250 ml de méthanol et on agite pendant 5 minutes. Le précipité en suspension est ensuite filtré sur Verre fritté 3. Le gâteau obtenu est ensuite lavé par 100 ml de méthanol. Le solide humide jaune clair est essoré puis séché sous pression réduite (6 kPa), à une température voisine de 40°C. Après 18 heures de séchage, on obtient 2,51 g d'héparine dépolymérisée sel de sodium dans la célite (5 g). Le rendement obtenu est de 64 %.

### Saponification :

Dans un erlenmeyer de 50 ml, on introduit 2,5 g (0,0038 mole) de l'héparine dépolymérisée brute, sel de sodium dans la célite (5 g) obtenue précédemment et 17 ml d'eau. La suspension est filtrée sur verre fritté 3 et rincé par 2 fois 5 ml d'eau. Le filtrat obtenu est chargé dans un erlenmeyer de 150 ml. Sous agitation magnétique, on introduit 0,4 ml (0,004 mole) de lessive de soude à 30 %, à une température voisine de 5°C. Après addition, le mélange est agité pendant 2 heures. On neutralise la solution par ajout d'HCl 1N et on ajoute 3 g de chlorure de sodium. Après dissolution on ajoute au milieu réactionnel 21 ml de méthanol. Après 15 minutes d'agitation on ajoute 44 ml de méthanol puis on agite pendant une heure. L'agitation est alors arrêtée et on laisse sédimenter la suspension pendant 45 minutes à une température voisine de 5°C. Le surnageant est ensuite prélevé puis écarté (90 ml). Sur le précipité sédimenté, on ajoute 90 ml de méthanol et on agite pendant 5 minutes. On laisse re-sédimenter le précipité 20 minutes environ. Le surnageant est prélevé et écarté (80 ml). Sur le précipité sédimenté, on ajoute 80 ml de méthanol et on agite pendant 5 minutes. Le précipité en suspension est ensuite filtré sur verre fritté 3. Le gâteau obtenu est ensuite lavé par 50 ml de méthanol. Le solide humide est essoré puis séché sous pression réduite (6 kPa), à une température voisine de 40°C. Après 18 heures de séchage, on obtient 1,31 g d'héparine dépolymérisée brute (sel de sodium). Le rendement obtenu est de 66 %.

### Purification :

Dans un erlenmeyer de 50 ml, on introduit 1,3 g d'héparine dépolymérisée brute obtenue précédemment et 13 ml d'eau distillée. Le mélange est porté à 40°C sous agitation magnétique. Par addition de soude 1N, on porte le pH à 9,7 ± 0,1. Le milieu réactionnel est filtré sur membrane 0,45 µm et on ajoute 0,07 ml d'une solution aqueuse de peroxyde d'hydrogène à 30 %. Après environ 2 heures d'agitation, à une température voisine de 20°C le mélange est neutralisé par ajout d'HCl à 1 N, puis on ajoute 2 g de chlorure de sodium. La solution est ensuite filtrée sur membrane 0,45 µm puis on coule 14 ml de méthanol. La solution est ensuite refroidie à 10°C et agité pendant environ 15 minutes. On ajoute alors 36 ml de méthanol puis on agite une heure. L'agitation est alors arrêtée et on laisse sédimenter la suspension pendant 15 minutes environ. Le surnageant est ensuite prélevé puis écarté (50 ml). Sur le précipité sédimenté, on ajoute 50 ml de méthanol et on agite pendant 5 minutes. On laisse re-sédimenter le précipité 25 minutes environ. Le surnageant est prélevé et écarté (50 ml). Le précipité en suspension est ensuite filtré sur verre fritté 3. Le gâteau blanc obtenu est ensuite lavé par 50 ml de méthanol. Le solide humide est essoré puis séché sous pression réduite (6 kPa), à une température voisine de 40°C. Après 18 heures de séchage, on obtient 1,13 g d'héparine dépolymérisée pure (sel de sodium). Le rendement obtenu est de 87 %.
Les caractéristiques de l'héparine dépolymérisée ainsi obtenue sont les suivantes :
Poids moléculaire moyen : 2600 Daltons
Activité anti-Xa : 177 UI/mg
Activité anti-IIa : 1,5 UI/mg
Rapport activité anti-Xa/activité anti-IIa : 118

### EXEMPLE 2

### Dépolymérisation et transformation en sel de sodium (0,2 %)

Dans un erlenmeyer A, on introduit 70 ml de dichlorométhane. Sous agitation et sous pression d'azote, on charge lentement 10 g (0,006 mole) d'ester benzylique d'héparine (taux d'estérification : 75 %, sel de benzéthonium) obtenu comme décrit dans l'exemple A. La teneur en eau du milieu réactionnel est ajustée à 0,2 %. Après dissolution complète, on ajoute 1,75 ml (0,006 mole) de 2-tert-butylimino-2-diethylamino-1,3-diméthylperhydro-1,2,3-diaza-phosphorine. On agite pendant 24 heures à une température voisine de 20°C. Pendant ce temps, on prépare dans un erlenmeyer B, une solution de 30 g d'acétate de sodium anhydre dans 300 ml de méthanol. Après dissolution complète on ajoute à la solution 5 g de célite Hyflo supercel. Sous agitation magnétique, le mélange réactionnel de l'erlenmeyer A est coulé en 1 minute 30 secondes dans la solution méthanolique d'acétate de sodium à une température voisine de 5°C. Après 5 minutes d'agitation on laisse décanter la suspension pendant 2 heures. La partie limpide du surnageant est prélevée et écartée (220 ml). Sur le précipité sédimenté, on ajoute 220 ml de méthanol et on agite pendant 5 minutes. On laisse re-sédimenter le précipité 2 heures environ. Le surnageant est prélevé et écarté (230 ml). Sur le précipité sédimenté, on ajoute 230 ml de méthanol. Le précipité en suspension est ensuite filtré sur verre fritté 3. Le gâteau obtenu est ensuite lavé par 150 ml de méthanol. Le solide humide jaune clair est essoré puis séché sous pression réduite (6 kPa), à une température voisine de 40°C. Après 18 heures de séchage, on obtient 2,63 g d'héparine dépolymérisée brute dans la célite (5 g). Le rendement obtenu est de 67 %.

### Saponification :

Dans un erlenmeyer de 50 ml, on introduit 2,5 g (0,0038 mole) de l'héparine dépolymérisée sel de sodium dans la célite (5 g) obtenue précédemment et 18 ml d'eau. La suspension est filtrée sur verre fritté 3 et rincé par 2 fois 5 ml d'eau. Le filtrat obtenu est chargé dans un erlenmeyer de 150 ml. Sous agitation magnétique, on introduit 0,4 ml (0,004 mole) de lessive de soude à 30 %, à une température voisine de 5°C. Après addition, le mélange est agité pendant 2 heures. On neutralise la solution par ajout d'HCl 1N et on ajoute 2 g de chlorure de sodium. On coule 14 ml de méthanol dans le mélange réactionnel. Après 15 minutes d'agitation on ajoute 36 ml de méthanol puis on agite pendant une heure. L'agitation est alors arrêtée et on laisse sédimenter la suspension pendant 45 minutes à une température voisine de 5°C. Le surnageant est ensuite prélevé puis écarté (80 ml). Sur le précipité sédimenté, on ajoute 80 ml de méthanol et on agite pendant 5 minutes. Le précipité en suspension est ensuite filtré sur verre fritté 3. Le gâteau obtenu est ensuite lavé par 50 ml de méthanol. Le solide humide est essoré puis séché sous pression réduite (6 kPa), à une température voisine de 40°C. Après 48 heures de séchage, on obtient 2,3 g d'héparine dépolymérisée brute (sel de sodium). Le rendement obtenu est de 65 %.

### Purification :

Dans un erlenmeyer de 50 ml, on introduit 1,4 g d'héparine dépolymérisée brute obtenue précédemment et 15 ml d'eau distillée. Le mélange est porté à 40°C sous agitation magnétique. Par addition de soude 1N, on porte le pH à 9,7 ± 0,1. Le milieu réactionnel est filtré sur membrane 0,45 µm et on ajoute 0,07 ml d'une solution aqueuse de peroxyde d'hydrogène à 30 %. Après environ 2 heures d'agitation, à une température voisine de 40°C le mélange est refroidit à une température voisine de 20°C puis neutralisé par ajout d'HCl 1N. on ajoute au milieu réactionnel 2 g de chlorure de sodium. La solution est ensuite filtrée sur membrane 0,45 µm puis on coule 14 ml de méthanol. La solution est ensuite refroidit à 10°C et agité 15 minutes. On ajoute alors 36 ml de méthanol puis on agite une heure. L'agitation est alors arrêtée et on laisse sédimenter la suspension pendant 15 minutes. Le surnageant est ensuite prélevé puis écarté (40 ml). Sur le précipité sédimenté (aspect de bouillie), on ajoute 40 ml de méthanol et on agite pendant 5 minutes. On laisse re-sédimenter le précipité 20 minutes environ. Le surnageant est prélevé et écarté (50 ml). Le précipité en suspension est ensuite filtré sur verre fritté 3. Le gâteau blanc obtenu est ensuite lavé par 50 ml de méthanol. Le solide humide est essoré puis séché sous pression réduite (6 kPa), à une température voisine de 40°C. Après 18 heures de séchage, on obtient 1,2 g d'héparine dépolymérisée pure (sel de sodium). Le rendement obtenu est de 86 %.

Les caractéristiques de l'héparine dépolymérisée ainsi obtenue sont les suivantes :
Poids moléculaire moyen : 2650 Daltons
Activité anti-Xa : 161 UI/mg
Activité anti-IIa : 1,4 UI/mg
Rapport activité anti-Xa/activité anti-IIa : 115

### EXEMPLE de référence 3

### Dépolymérisation et transformation en sel de sodium (0,3 % d'eau)

Dans un erlenmeyer A, on introduit 70 ml de dichlorométhane. Sous agitation et sous pression d'azote, on charge 10 g (0,006 mole) d'ester benzylique d'héparine (taux d'estérification : 75 %, sel de benzéthonium) obtenu comme décrit dans l'exemple A. La teneur en eau du milieu réactionnel est ajustée 0,3 %. Après dissolution complète, on ajoute 1,75 ml (0,006 mole) de 2-tert-butylimino-2-diethylamino-1,3-diméthylperhydro-1,2,3-diaza-phosphorine. On agite à une température voisine de 20°C, pendant 24 heures. Pendant ce temps, on prépare dans un erlenmeyer B, une solution de 30 g d'acétate de sodium anhydre dans 300 ml de méthanol. Après dissolution complète, on ajoute à la solution 5 g de célite Hyflo supercel. Sous agitation magnétique, le mélange réactionnel de l'erlenmeyer A est coulé en 1 minute 30 secondes dans la solution méthanolique d'acétate de sodium à une température voisine de 5°C. Après 5 minutes d'agitation, on laisse décanter la suspension pendant 1 heure 10 minutes. La partie limpide du surnageant est prélevée et écartée (220 ml). Sur le précipité sédimenté, on ajoute 220 ml de méthanol et on agite pendant 5 minutes. Le précipité en suspension est ensuite filtré sur verre fritté 3. Le gâteau obtenu est ensuite lavé par 100 ml de méthanol. Le solide humide est essoré puis séché sous pression réduite (6 kPa), à une température voisine de 40°C. Après 18 heures de séchage, on obtient 2,57 g d'héparine dépolymérisée brute, sel de sodium dans la célite (5 g). Le rendement obtenu est de 66 %.

### Saponification :

Dans un erlenmeyer de 50 ml, on introduit 2,5 g (0,0038 mole) de l'héparine dépolymérisée brute, sel de sodium dans la célite (5 g) obtenue précédemment et 18 ml d'eau. La suspension est filtrée sur verre fritté 3 et rincé par 2 fois 5 ml d'eau. Le filtrat obtenu est chargé dans un erlenmeyer de 150 ml. Sous agitation magnétique, on introduit 0,4 ml (0,004 mole) de lessive de soude à 30 %, à une température voisine de 5°C. Après addition, le mélange est agité pendant 2 heures. On neutralise la solution par ajout d'HCl 1N et on ajoute 3 g de chlorure de sodium. On coule 15 ml de méthanol dans le mélange réactionnel. Après 15 minutes d'agitation, on ajoute 36 ml de méthanol puis on agite pendant une heure. L'agitation est alors arrêtée et on laisse sédimenter la suspension pendant 1 heure. Le surnageant est ensuite prélevé puis écarté (70 ml). Sur le précipité sédimenté, on ajoute 70 ml de méthanol et on agite pendant 5 minutes. Le précipité en suspension est ensuite filtré sur verre fritté 3. Le gâteau obtenu est ensuite lavé par 50 ml de méthanol. Le solide humide est essoré puis séché sous pression réduite (6 kPa), à une température voisine de 40°C. Après 48 heures de séchage, on obtient 1,42 g d'héparine dépolymérisée brute (sel de sodium). Le rendement obtenu est de 62 %.

### Purification :

Dans un erlenmeyer de 50 ml, on introduit 1,4 g d'héparine dépolymérisée brute obtenue précédemment et 14 ml d'eau distillée. Le mélange est porté à 40°C sous agitation magnétique. Par addition de soude 1N, on porte le pH à 9,7 ± 0,1. Le milieu réactionnel est filtré sur membrane 0,45 µm et on ajoute 0,07 ml d'une solution aqueuse de peroxyde d'hydrogène à 30 %. Après environ 2 heures d'agitation, à une température voisine de 20°C le mélange est neutralisé par ajout d'HCl 1N, puis on ajoute 2 g de chlorure de sodium. Après dissolution la solution est ensuite filtrée sur membrane 0,45 µm puis on coule 14 ml de méthanol. Le filtrat est ensuite refroidi à 10°C et agité 15 minutes. On ajoute alors 36 ml de méthanol puis on agite environ une heure. L'agitation est alors arrêtée et on laisse sédimenter la suspension pendant 40 minutes. Le surnageant est ensuite prélevé puis écarté (50 ml). Sur le précipité sédimenté (aspect de bouillie), on ajoute 50 ml de méthanol et on agite pendant 5 minutes. On laisse re-sédimenter le précipité 25 minutes environ. Le précipité en suspension est ensuite filtré sur verre fritté 3. Le gâteau blanc obtenu est ensuite lavé par 50 ml de méthanol. Le solide humide est essoré puis séché sous pression réduite (6 kPa), à une température voisine de 40°C. Après 18 heures de séchage, on obtient 1,24 g d'héparine dépolymérisée pure (sel de sodium). Le rendement obtenu est de 89 %.

Les caractéristiques de l'héparine dépolymérisée ainsi obtenue sont les suivantes :
Poids moléculaire moyen : 2400 Daltons
Activité anti-Xa : 132 UI/mg
Activité anti-IIa : 1,4 UI/mg
Rapport activité anti-Xa/activité anti-IIa : 94

### EXEMPLE de référence 4

### Dépolymérisation et transformation en sel de sodium ( 0,4 % en eau) :

Dans un erlenmeyer A, on introduit 70 ml de dichlorométhane. Sous agitation, on charge lentement 10 g (0,006 mole) d'ester benzylique d'héparine (taux d'estérification : 75 %, sel de benzéthonium) obtenu comme décrit dans l'exemple A. La teneur en eau du milieu réactionnel est ajusté à 0,4 %. La solution est chauffée à 30°C sous azote. Après dissolution complète, on revient à une température voisine de 20°C puis on ajoute 1,75 ml (0,006 mole) de 2-tert-butylimino-2-diethylamino-1,3-diméthylperhydro-1,2,3-diaza-phosphorine. On agite à une température voisine de 20°C, pendant 24 heures. Pendant ce temps, on prépare dans un erlenmeyer B, une solution de 30 g d'acétate de sodium anhydre dans 300 ml de méthanol. Après dissolution complète, on ajoute à la solution 5 g de célite Hyflo supercel. Sous agitation magnétique, le mélange réactionnel de l'erlenmeyer A est coulé en environ 1 minute 30 secondes dans la solution méthanolique d'acétate de sodium à une température voisine de 5°C. Après 5 minutes d'agitation, on laisse décanter la suspension pendant 2 heures. La partie limpide du surnageant est prélevée et écartée (80 ml). Sur le précipité sédimenté, on ajoute 80 ml de méthanol et on agite pendant 5 minutes. On laisse re-sédimenter le précipité 1 heure environ. Le surnageant est prélevé et écarté (80 ml). Sur le précipité sédimenté, on ajoute 80 ml de méthanol et on agite pendant 5 minutes. Le précipité en suspension est ensuite filtré sur verre fritté 3. Le gâteau obtenu est ensuite lavé par 150 ml de méthanol. Le solide humide jaune clair est essoré puis séché sous pression réduite (6 kPa), à une température voisine de 40°C. Après 18 heures de séchage, on obtient 3,25 g d'héparine dépolymérisée brute, sel de sodium dans la célite (5 g). Le rendement obtenu est de 83 %.

### Saponification :

Dans un erlenmeyer de 50 ml, on introduit 3,1 g (0,0018 mole) de l'héparine dépolymérisée brute, sel de sodium dans la celite (10 g) obtenue précédemment et 21 ml d'eau. La suspension est filtrée sur verre fritté 3 et rincé par 2 fois 6 ml d'eau. Le filtrat obtenu est chargé dans un erlenmeyer de 150 ml. Sous agitation magnétique, on introduit 0,7 ml (0,007 mole) de lessive de soude à 30 %, à une température voisine de 5°C. Après addition, le mélange est agité pendant 2 heures. On neutralise la solution par ajout d'HCl 1N et on ajoute 4 g de chlorure de sodium. On coule 28 ml de méthanol dans le mélange réactionnel. Après 15 minutes d'agitation, on ajoute 72 ml de méthanol puis on agite pendant une heure. L'agitation est alors arrêtée et on laisse sédimenter la suspension pendant 1 heure. Le surnageant est ensuite prélevé puis écarté (90 ml). Sur le précipité sédimenté, on ajoute 90 ml de méthanol et on agite pendant 5 minutes. On laisse re-sédimenter le précipité 20 minutes environ. Le surnageant est prélevé et écarté (90 ml). Sur le précipité sédimenté, on ajoute 90 ml de méthanol et on agite pendant 5 minutes. Le précipité en suspension est ensuite filtré sur verre fritté 3. Le gâteau obtenu est ensuite lavé par 50 ml de méthanol. Le solide humide est essoré puis séché sous pression réduite (6 kPa), à une température voisine de 40°C. Après 48 heures de séchage, on obtient 1,9 g d'héparine dépolymérisée brute (sel de sodium). Le rendement obtenu est de 67 %.

### Purification :

Dans un erlenmeyer de 50 ml, on introduit 1,9 g d'héparine dépolymérisée brute obtenue précédemment et 19 ml d'eau distillée. Le mélange est porté à 40°C sous agitation magnétique. Par addition de soude 1N, on porte le pH à 9,7 ± 0,1. Le milieu réactionnel est filtré sur membrane 0,45 µm et on ajoute 0,1 ml d'une solution aqueuse de peroxyde d'hydrogène à 30 %. Après environ 2 heures d'agitation, à une température voisine de 20°C le mélange est neutralisé par ajout d'HCl 1N, puis on ajoute 2 g de chlorure de sodium. La solution est ensuite filtrée sur membrane 0,45 µm puis on coule 14 ml de méthanol et on agite 15 minutes. On ajoute alors 36 ml de méthanol puis on agite une heure. L'agitation est alors arrêtée et on laisse sédimenter la suspension pendant 15 minutes. Le surnageant est ensuite prélevé puis écarté (40 ml). Sur le précipité sédimenté, on ajoute 40 ml de méthanol et on agite pendant 5 minutes. On laisse re-sédimenter le précipité 20 minutes environ. Le surnageant est prélevé et écarté (50 ml). Sur le précipité sédimenté, on ajoute 500 ml de méthanol et on agite pendant 5 minutes. On laisse re-sédimenter le précipité 20 minutes environ. La suspension est ensuite filtrée sur verre fritté 3. Le gâteau blanc obtenu est ensuite lavé par 50 ml de méthanol. Le solide humide est essoré puis séché sous pression réduite (6 kPa), à une température voisine de 40°C. Après 72 heures de séchage, on obtient 1,56 g d'héparine dépolymérisée pure (sel de sodium). Le rendement obtenu est de 82 %.

Les caractéristiques de l'héparine dépolymérisée ainsi obtenue sont les suivantes :
Poids moléculaire moyen : 2350 Daltons
Activité anti-Xa : 122 UI/mg
Activité anti-IIa : 1.3 UI/mg
Rapport activité anti-Xa/activité anti-IIa : 94

### EXEMPLE de référence 5

### Dépolymérisation et transformation en sel de sodium (0,57 % d'eau) :

Dans un erlenmeyer A, on introduit 140 ml de dichlorométhane. Sous agitation, on charge lentement 20 g (0,019 mole) d'ester benzylique d'héparine (taux d'estérification : 75 %, sel de benzéthonium) obtenu comme décrit dans l'exemple A. La teneur en eau du milieu réactionnel est ajustée à 0,57 %. Après dissolution complète, on ajoute 3,5 ml (0,012 mole) de 2-tert-butylimino-2-diethylamino-1,3-diméthyl-perhydro-1,2,3-diaza-phosphorine. On agite à une température voisine de 20°C, pendant 24 heures. Pendant ce temps, on prépare dans un erlenmeyer B, une solution de 60 g d'acétate de sodium anhydre dans 600 ml de méthanol. Après dissolution complète, on ajoute à la solution 10 g de célite Hyflo supercel. Sous agitation magnétique, le mélange réactionnel de l'erlenmeyer A est coulé en 1 minute 30 secondes dans la solution méthanolique d'acétate de sodium à une température voisine de 4°C. Après 5 minutes d'agitation on laisse décanter la suspension pendant 30 minutes. La partie limpide du surnageant est prélevée et écartée (400 ml). Sur le précipité sédimenté, on ajoute 400 ml de méthanol et on agite pendant 5 minutes. On laisse re-sédimenter le précipité 1 heure environ. Le surnageant est prélevé et écarté (420 ml). Sur le précipité sédimenté, on ajoute 420 ml de méthanol et on agite pendant 5 minutes. La suspension est ensuite filtré sur verre fritté 3. Le gâteau obtenu est ensuite lavé par 200 ml de méthanol. Le solide humide jaune clair est essoré puis séché sous pression réduite (6 kPa), à une température voisine de 50°C. Après 18 heures de séchage, on obtient 6,66 g d'héparine dépolymérisée brute, sel de sodium dans la célite (10 g). Le rendement obtenu est de 85 %.

### Saponification :

Dans un erlenmeyer de 50 ml, on introduit 6,66 g (0,0101 mole) de l'héparine dépolymérisée brute, sel de sodium dans la célite (10 g) obtenue précédemment et 47 ml d'eau. La suspension est filtrée sur verre fritté 3 et rincé par 2 fois 15 ml d'eau. Le filtrat obtenu est chargé dans un erlenmeyer de 150 ml. Sous agitation magnétique, on introduit 1,1 ml (0,011 mole) de lessive de soude à 30 %, à une température voisine de 5°C. Après addition, le mélange est agité pendant 2 heures. On neutralise la solution par ajout d'HCl 1N et on ajoute 9,5 g de chlorure de sodium. On ajoute au milieu réactionnel 66 ml de méthanol. Après 15 minutes d'agitation, on ajoute 171 ml de méthanol puis on agite pendant une heure. L'agitation est alors arrêtée et on laisse sédimenter la suspension pendant 3/4 d'heure à une température voisine de 5°C. Le surnageant est ensuite prélevé puis écarté (160 ml). Sur le précipité sédimenté, on ajoute 160 ml de méthanol et on agite pendant 5 minutes. On laisse re-sédimenter le précipité 20 minutes environ. Le surnageant est prélevé et écarté (180 ml). Sur le précipité sédimenté, on ajoute 180 ml de méthanol et on agite pendant 5 minutes. La suspension est ensuite filtrée sur verre fritté 3. Le gâteau obtenu est ensuite lavé par 2 fois 50 ml de méthanol. Le solide humide est essoré puis séché sous pression réduite (6 kPa), à une température voisine de 40°C. Après 18 heures de séchage, on obtient 4,53 g d'héparine dépolymérisée brute (sel de sodium). Le rendement obtenu est de 74 %.

### Purification :

Dans un erlenmeyer de 100 ml, on introduit 4,53 g d'héparine dépolymérisée brute obtenue précédemment et 45 ml d'eau distillée. Le mélange est porté à 40°C sous agitation magnétique. Par addition de soude 1N, on porte le pH à 9,7 ± 0,1. Le milieu réactionnel est filtré sur membrane 0,45 µm et on ajoute 0,25 ml d'une solution aqueuse de peroxyde d'hydrogène à 30 %. Après environ 2 heures d'agitation, à une température voisine de 20°C le mélange est neutralisé par ajout d'HCl à 1 N, puis on ajoute 5,5 g de chlorure de sodium. La solution est ensuite filtrée sur membrane 0,45 µm puis on coule 38 ml de méthanol à une température voisine de 10°C. La solution est ensuite amenée à 20°C et agité 15 minutes. On ajoute alors 100 ml de méthanol puis on agite une heure. L'agitation est alors arrêtée et on laisse sédimenter la suspension pendant 20 minutes. Le surnageant est ensuite prélevé puis écarté (90 ml). Sur le précipité sédimenté, on ajoute 90 ml de méthanol et on agite pendant 5 minutes. On laisse re-sédimenter le précipité 25 minutes environ. Le surnageant est prélevé et écarté (100 ml). Le précipité en suspension est ensuite filtré sur verre fritté 3. Le gâteau blanc obtenu est ensuite lavé par 50 ml de méthanol. Le solide humide est essoré puis séché sous pression réduite (6 kPa), à une température voisine de 50°C. Après 18 heures de séchage, on obtient 3,7 g d'héparine dépolymérisée pure (sel de sodium). Le rendement obtenu est de 82 %. Les caractéristiques de l'héparine dépolymérisée ainsi obtenue sont les suivantes :
Poids moléculaire moyen : 2200 Daltons
Activité anti-Xa : 120 UI/mg
Activité anti-IIa : 1.4 UI/mg
Rapport activité anti-Xa/activité anti-IIa : 86

### EXEMPLE de référence 6

### Dépolymérisation et transformation en sel de sodium (1,8 % d'eau) :

Dans un erlenmeyer A, on introduit 140 ml de dichlorométhane. Sous agitation, on charge lentement 20 g (0,019 mole) d'ester benzylique d'héparine (taux d'estérification : 75 %, sel de benzéthonium) obtenu comme décrit dans l'exemple A. La teneur en eau du milieu réactionnel est ajustée à 1,8 %. Après dissolution complète, on ajoute 3,5 ml (0,012 mole) de 2-tert-butylimino-2-diethylamino-1,3-diméthylperhydro-1,2,3-diaza-phosphorine. On agite à une température voisine de 20°C, pendant 24 heures. Pendant ce temps, on prépare dans un erlenmeyer B, une solution de 60 g d'acétate de sodium anhydre dans 600 ml de méthanol. Après dissolution complète, on ajoute à la solution 10 g de célite Hyflo supercel. Sous agitation magnétique, le mélange réactionnel de l'erlenmeyer A est coulé en 1 minute 30 secondes dans la solution méthanolique d'acétate de sodium à une température voisine de 4°C. Après 5 minutes d'agitation, on laisse décanter la suspension pendant 30 minutes. La partie limpide du surnageant est prélevée et écartée (400 ml). Sur le précipité sédimenté, on ajoute 400 ml de méthanol et on agite pendant 5 minutes. On laisse re-sédimenter le précipité 1 heure environ. Le surnageant est prélevé et écarté (420 ml). Sur le précipité sédimenté, on ajoute 420 ml de méthanol et on agite pendant 5 minutes. Le précipité en suspension est ensuite filtré sur verre fritté 3. Le gâteau obtenu est ensuite lavé par 200 ml de méthanol. Le solide humide jaune clair est essoré puis séché sous pression réduite (6 kPa), à une température voisine de 50°C. Après 18 heures de séchage, on obtient 7,54 g d'héparine dépolymérisée brute, sel de sodium dans la celite (10 g). Le rendement obtenu est de 96 %.

### Saponification :

Dans un erlenmeyer de 50 ml, on introduit 7,54 g (0,0101 mole) de l'héparine dépolymérisée brute, sel de sodium dans la célite (10 g) obtenue précédemment et 53 ml d'eau. La solution est filtrée sur verre fritté 3 et rincé par 2 fois 15 ml d'eau. Le filtrat obtenu est chargé dans un erlenmeyer de 150 ml. Sous agitation magnétique, on introduit 1,25 ml (0,012 mole) de lessive de soude à 30 %, à une température voisine de 4°C. Après addition, le mélange est agité pendant 2 heures. On neutralise la solution par ajout d'HCl 1N et on ajoute 10,5 g de chlorure de sodium. On ajoute au milieu réactionnel 70 ml de méthanol. Après 15 minutes d'agitation, on ajoute 190 ml de méthanol puis on agite pendant une heure. L'agitation est alors arrêtée et on laisse sédimenter la suspension pendant 3/4 d'heure à une température voisine de 4°C. Le surnageant est ensuite prélevé puis écarté (180 ml). Sur le précipité sédimenté, on ajoute 180 ml de méthanol et on agite pendant 5 minutes. On laisse re-sédimenter le précipité 20 minutes environ. Le surnageant est prélevé et écarté (180 ml). Sur le précipité sédimenté, on ajoute 180 ml de méthanol et on agite pendant 5 minutes. Le précipité en suspension est ensuite filtré sur verre fritté 3. Le gâteau obtenu est ensuite lavé par 2 fois 50 ml de méthanol. Le solide humide est essoré puis séché sous pression réduite (6 kPa), à une température voisine de 50°C. Après 18 heures de séchage, on obtient 5,53 g d'héparine dépolymérisée brute (sel de sodium). Le rendement obtenu est de 80 %.

### Purification :

Dans un erlenmeyer de 100 ml, on introduit 5,53 g d'héparine dépolymérisée brute obtenue précédemment et 55 ml d'eau distillée. Le mélange est porté à 40°C sous agitation magnétique. Par addition de soude 1N, on porte le pH à 9,7 ± 0,1. Le milieu réactionnel est filtré sur membrane 0,45 µm et on ajoute 0,31 ml d'une solution aqueuse de peroxyde d'hydrogène à 30 %. Après environ 2 heures d'agitation, à une température voisine de 20°C le mélange est neutralisé par ajout d'HCl 1N, puis on ajoute 7 g de chlorure de sodium. La solution est ensuite filtrée sur membrane 0,45 µm puis on coule 49 ml de méthanol à une température voisine de 10°C. La solution est ensuite amenée à 20°C et agité 15 minutes. On ajoute alors 126 ml de méthanol puis on agite une heure. L'agitation est alors arrêtée et on laisse sédimenter la suspension pendant 20 minutes. Le surnageant est ensuite prélevé puis écarté (105 ml). Sur le précipité sédimenté, on ajoute 105 ml de méthanol et on agite pendant 5 minutes. On laisse re-sédimenter le précipité 25 minutes environ. Le surnageant est prélevé et écarté (110 ml). Le précipité en suspension est ensuite filtré sur verre fritté 3. Le gâteau blanc obtenu est ensuite lavé par 50 ml de méthanol. Le solide humide est essoré puis séché sous pression réduite (6 kPa), à une température voisine de 55°C. Après 18 heures de séchage, on obtient 4,53 g d'héparine dépolymérisée pure (sel de sodium). Le rendement obtenu est de 82 %.

Les caractéristiques de l'héparine dépolymérisée ainsi obtenue sont les suivantes :
Poids moléculaire moyen : 2600 Daltons
Activité anti-Xa : 105 UI/mg
Activité anti-IIa : 3.1 UI/mg
Rapport activité anti-Xa/activité anti-IIa : 34

### EXEMPLE de référence 7

### Dépolymérisation et transformation en sel de sodium (2,5 % d'eau) :

Dans un erlenmeyer A, on introduit 140 ml de dichlorométhane. Sous agitation, on charge lentement 20 g (0,019 mole) d'ester benzylique d'héparine (taux d'estérification : 75 %, sel de benzéthonium) obtenu comme décrit dans l'exemple A. La teneur en eau du milieu réactionnel est ajustée à 2,5 %. Après dissolution complète, on ajoute 3,5 ml (0,012 mole) de 2-tert-butylimino-2-diethylamino-1,3-diméthylperhydro-1,2,3-diaza-phosphorine. On agite à une température voisine de 20°C, pendant 24 heures. Pendant ce temps, on prépare dans un erlenmeyer B, une solution de 60 g d'acétate de sodium anhydre dans 600 ml de méthanol. Après dissolution complète, on ajoute à la solution 10 g de célite Hyflo supercel. Sous agitation magnétique, le mélange réactionnel de l'erlenmeyer A est coulé en 1 minute 30 secondes dans la solution méthanolique d'acétate de sodium à une température voisine de 4°C. Après 5 minutes d'agitation, on laisse décanter la suspension pendant 1 heure. La partie limpide du surnageant est prélevée et écartée (400 ml). Sur le précipité sédimenté, on ajoute 400 ml de méthanol et on agite pendant 5 minutes. On laisse re-sédimenter le précipité 30 minutes environ. La partie limpide du surnageant est prélevée et écartée (400 ml). Le précipité en suspension est ensuite filtré sur verre fritté 3. Le gâteau obtenu est ensuite lavé par 200 ml de méthanol. Le solide humide jaune clair est essoré puis séché sous pression réduite (6 kPa), à une température voisine de 50°C. Après 18 heures de séchage, on obtient 7,78 g d'héparine dépolymérisée brute, sel de sodium dans la célite (10 g). Le rendement obtenu est de 99,6 %.

### Saponification :

Dans un erlenmeyer de 50 ml, on introduit 7,78 g (0,0119 mole) de l'héparine dépolymérisée brute, sel de sodium dans la célite (10 g) obtenue précédemment et 79 ml d'eau. La suspension est filtrée sur verre fritté 3 et rincé par 2 fois 15 ml d'eau. Le filtrat obtenu est chargé dans un erlenmeyer de 150 ml. Sous agitation magnétique, on introduit 1,3 ml (0,012 mole) de lessive de soude à 30 %, à une température voisine de 4°C. Après addition, le mélange est agité pendant 2 heures. On neutralise la solution par ajout d'HCl 1N et on ajoute 10 g de chlorure de sodium. On ajoute au milieu réactionnel 60 ml de méthanol. Après 15 minutes d'agitation, on ajoute 190 ml de méthanol puis on agite pendant une heure. L'agitation est alors arrêtée et on laisse sédimenter la suspension pendant 3/4 d'heure à une température voisine de 4°C. Le surnageant est ensuite prélevé puis écarté (180 ml). Sur le précipité sédimenté, on ajoute 180 ml de méthanol et on agite pendant 5 minutes. On laisse re-sédimenter le précipité 20 minutes environ. Le surnageant est prélevé et écarté (180 ml). Sur le précipité sédimenté, on ajoute 180 ml de méthanol et on agite pendant 5 minutes. Le précipité en suspension est ensuite filtré sur verre fritté 3. Le gâteau obtenu est ensuite lavé par 2 fois 50 ml de méthanol. Le solide humide est essoré puis séché sous pression réduite (6 kPa), à une température voisine de 50°C. Après 18 heures de séchage, on obtient 5,87 g d'héparine dépolymérisée brute (sel de sodium). Le rendement obtenu est de 82 %.

### Purification :

Dans un erlenmeyer de 100 ml, on introduit 5,87 g d'héparine dépolymérisée brute obtenue précédemment et 59 ml d'eau distillée. Le mélange est porté à 40°C sous agitation magnétique. Par addition de soude 1N, on porte le pH à 9,7 ± 0,1. Le milieu réactionnel est filtré sur membrane 0,45 µm et on ajoute 0,34 ml d'une solution aqueuse de peroxyde d'hydrogène à 30 %. Après environ 2 heures d'agitation, à une température voisine de 20°C le mélange est neutralisé par ajout d'HCl à 1 N, puis on ajoute 7 g de chlorure de sodium. La solution est ensuite filtrée sur membrane 0,45 µm puis on coule 49 ml de méthanol à une température voisine de 10°C. La solution est ensuite amenée à 20°C et agité 15 minutes. On ajoute alors 126 ml de méthanol puis on agite une heure, L'agitation est alors arrêtée et on laisse sédimenter la suspension pendant 20 minutes. Le surnageant est ensuite prélevé puis écarté (105 ml). Sur le précipité sédimenté, on ajoute 105 ml de méthanol et on agite pendant 5 minutes. On laisse re-sédimenter le précipité 25 minutes environ. Le surnageant est prélevé et écarté (110 ml). Le précipité en suspension est ensuite filtré sur verre fritté 3. Le gâteau blanc obtenu est ensuite lavé par 50 ml de méthanol. Le solide humide est essoré puis séché sous pression réduite (6 kPa), à une température voisine de 55°C. Après 18 heures de séchage, on obtient 5,21 g d'héparine dépolymérisée pure (sel de sodium). Le rendement obtenu est de 89 %.

Les caractéristiques de l'héparine dépolymérisée ainsi obtenue sont les suivantes :
Poids moléculaire moyen : 3550 Daltons
Activité anti-Xa : 99 UI/mg
Activité anti-IIa : 13,4 UI/mg
Rapport activité anti-Xa/activité anti-IIa : 7.4

### EXEMPLE de référence 8

### Dépolymérisation et transformation en sel de sodium (0,5 équivalents de base) :

Dans un erlenmeyer A, on introduit 140 ml de dichlorométhane. Sous agitation, on charge lentement 20 g (0,019 mole) d'ester benzylique d'héparine (taux d'estérification : 75 %, sel de benzéthonium) obtenu comme décrit dans l'exemple A. Après dissolution complète à une température voisine de 30°C et retour à une température d'environ 20°C, on ajoute 1,75 ml (0,006 mole) de 2-tert-butylimino-2-diethylamino-1,3-diméthylperhydro-1,2,3-diazaphosphorine. On agite à une température voisine de 20°C, pendant 24 heures. Pendant ce temps, on prépare dans un erlenmeyer B, une solution de 60 g d'acétate de sodium anhydre dans 600 ml de méthanol. Sous agitation magnétique, le mélange réactionnel de l'erlenmeyer A est coulé dans la solution méthanolique d'acétate de sodium à une température voisine de 4°C. Après 1 heure d'agitation on laisse décanter la suspension pendant 2 heures. La partie limpide du surnageant est prélevée et écartée (420 ml). Sur le précipité sédimenté, on ajoute 420 ml de méthanol et on agite pendant 30 minutes. On laisse re-sédimenter le précipité 18 heures environ. La partie limpide du surnageant est prélevée et écartée (400 ml). Sur le précipité sédimenté, on ajoute 400 ml de méthanol et on agite pendant 30 minutes. Le surnageant est prélevé et écarté (400 ml). Le précipité en suspension est ensuite filtré sur verre fritté 3. Le gâteau obtenu est ensuite lavé par 2 fois 100 ml de méthanol. Le solide humide jaune clair est essoré puis séché sous pression réduite (6 kPa), à une température voisine de 60°C. Après séchage, on obtient 5,7 g d'héparine dépolymérisée brute, sel de sodium. Le rendement obtenu est de 73 %.

### Saponification :

Dans un erlenmeyer de 100 ml, on introduit 5,7 g (0,0086 mole) de l'héparine dépolymérisée brute, sel de sodium obtenue précédemment et 53 ml d'eau. Sous agitation magnétique, on introduit 0,93 ml (0,009 mole) de lessive de soude à 30 %, à une température voisine de 4°C. Après addition, le mélange est agité pendant 2 heures. On neutralise la solution par ajout d'HCl 1N et on ajoute 6 g de chlorure de sodium. On ajoute au milieu réactionnel 42 ml de méthanol. Après 15 minutes d'agitation on ajoute 108 ml de méthanol puis on agite pendant 30 minutes. L'agitation est alors arrêtée et on laisse sédimenter la suspension pendant 30 minutes à une température voisine de 4°C. Le surnageant est ensuite prélevé puis écarté (180 ml). Sur le précipité sédimenté, on ajoute 180 ml de méthanol et on agite pendant 5 minutes. On laisse re-sédimenter le précipité 30 minutes environ. Le surnageant est prélevé et écarté (170 ml). Sur le précipité sédimenté, on ajoute 170 ml de méthanol et on agite pendant 30 minutes. Le précipité en suspension est ensuite filtré sur verre fritté 3. Le gâteau obtenu est ensuite lavé par 2 fois 30 ml de méthanol. Le solide humide est essoré puis séché sous pression réduite, à une température voisine de 60°C. Après séchage, on obtient 3,5 g d'héparine dépolymérisée brute (sel de sodium). Le rendement obtenu est de 67,4 %.

### Purification :

Dans un erlenmeyer de 100 ml, on introduit 3,5 g d'héparine dépolymérisée brute obtenue précédemment et 35 ml d'eau distillée. Le mélange est porté à 40°C sous agitation magnétique. Par addition de soude 1N, on porte le pH à 9,6 ± 0,1. Le milieu réactionnel est filtré sur membrane 0,45 µm et on ajoute 0,18 ml d'une solution aqueuse de peroxyde d'hydrogène à 30 %. Après environ 2 heures d'agitation, à une température voisine de 20°C le mélange est neutralisé par ajout d'HCl à 1N, puis on ajoute 3,6 g de chlorure de sodium. La solution est ensuite filtrée sur membrane 0,45 µm puis on coule 27 ml de méthanol à une température voisine de 10°C. La solution est ensuite amenée à 20°C et agité 15 minutes. On ajoute alors 65 ml de méthanol puis on agite 30 minutes. L'agitation est alors arrêtée et on laisse sédimenter la suspension pendant 30 minutes. Le surnageant est ensuite prélevé puis écarté (80 ml). Sur le précipité sédimenté, on ajoute 80 ml de méthanol et on agite pendant 30 minutes. On laisse re-sédimenter le précipité 30 minutes environ. Le surnageant est prélevé et écarté (70 ml). Sur le précipité sédimenté, on ajoute 70 ml de méthanol et on agite pendant 30 minutes. Le précipité en suspension est ensuite filtré sur verre fritté 3. Le gâteau blanc obtenu est ensuite lavé par 2 fois 30 ml de méthanol. Le solide humide est essoré puis séché sous pression réduite, à une température voisine de 60°C. Après séchage, on obtient 2,8 g d'héparine dépolymérisée pure (sel de sodium). Le rendement obtenu est de 80 %.

Les caractéristiques de l'héparine dépolymérisée ainsi obtenue sont les suivantes :
Poids moléculaire moyen : 2900 Daltons
Activité anti-Xa : 146,1 UI/mg
Activité anti-IIa : 5,1 UI/mg
Rapport activité anti-Xa/activité anti-IIa : 28,6

### EXEMPLE 9

### Dépolymérisation et transformation en sel de sodium (0,6 equivalents de base):

Dans un tricol A, on introduit 280 ml de dichlorométhane. Sous agitation, on charge lentement 40 g (0,024 mole) d'ester benzylique d'héparine (taux d'estérification : 75 %, sel de benzéthonium) obtenu comme décrit dans l'exemple A. Après dissolution complète à une température voisine de 30°C et retour à une température voisine de 20°C, on ajoute 4,2 ml (0,014 mole) de 2-tert-butylimino-2-diethylamino-1,3-diméthylperhydro-1,2,3-diaza-phosphorine. On agite à une température voisine de 20°C, pendant 24 heures. Pendant ce temps, on prépare dans un erlenmeyer B, une solution de 60 g d'acétate de sodium anhydre dans 600 ml de méthanol. Sous agitation magnétique, la moitié du mélange réactionnel du tricol A est coulé dans la solution méthanolique d'acétate de sodium à une température voisine de 4°C. Après 1 heure d'agitation on laisse décanter la suspension. La partie limpide du surnageant est prélevée et écartée (310 ml). Sur le précipité sédimenté, on ajoute 310 ml de méthanol et on agite pendant 1 heure. On laisse re-sédimenter le précipité 18 heures environ. La partie limpide du surnageant est prélevée et écartée (400 ml). Sur le précipité sédimenté, on ajoute 400 ml de méthanol et on agite pendant 1 heure. Le surnageant est prélevé et écarté (300 ml). Le précipité en suspension est ensuite filtré sur verre fritté 3. Le gâteau obtenu est ensuite lavé par 2 fois 100 ml de méthanol. Le solide humide jaune clair est essoré puis séché sous pression réduite, à une température voisine de 60°C. Après séchage, on obtient 6 g d'héparine dépolymérisée brute, sel de sodium. Le rendement obtenu est de 77 %.

### Saponification :

Dans un erlenmeyer de 250 ml, on introduit 6 g (0,0091 mole) de l'héparine dépolymérisée brute (sel de sodium) obtenue précédemment et 56 ml d'eau. Sous agitation magnétique, on introduit 1 ml (0,010 mole) de lessive de soude à 30 %, à une température voisine de 4°C. Après addition, le mélange est agité pendant 2 heures. On neutralise la solution par ajout d'HCl 1N et on ajoute 6,4 g de chlorure de sodium. On ajoute au milieu réactionnel 45 ml de méthanol. Après 15 minutes d'agitation on ajoute 115 ml de méthanol puis on agite pendant 30 minutes. L'agitation est alors arrêtée et on laisse sédimenter la suspension pendant 30 minutes à une température voisine de 4°C. Le surnageant est ensuite prélevé puis écarté (170 ml). Sur le précipité sédimenté, on ajoute 170 ml de méthanol et on agite pendant 30 minutes. On laisse re-sédimenter le précipité 30 minutes environ. Le surnageant est prélevé et écarté (140 ml). Sur le précipité sédimenté, on ajoute 140 ml de méthanol et on agite pendant 30 minutes. Le précipité en suspension est ensuite filtré sur verre fritté 3. Le gâteau obtenu est ensuite lavé par 2 fois 30 ml de méthanol. Le solide humide est essoré puis séché sous pression réduite, à une température voisine de 60°C. Après séchage, on obtient 3,6 g d'héparine dépolymérisée brute (sel de sodium). Le rendement obtenu est de 65,3 %.

### Purification :

Dans un erlenmeyer de 100 ml, on introduit 3,5 g d'héparine dépolymérisée brute obtenue précédemment et 35 ml d'eau distillée. Le mélange est porté à 40°C sous agitation magnétique. Par addition de soude 1N, on porte le pH à 9,6 ± 0,1. Le milieu réactionnel est filtré sur membrane 0,45 µm et on ajoute 0,18 ml d'une solution aqueuse de peroxyde d'hydrogène à 30 %. Après environ 2 heures d'agitation, à une température voisine de 20°C, le mélange est neutralisé par ajout d'HCl à 1N, puis on ajoute 3,5 g de chlorure de sodium. La solution est ensuite filtrée sur membrane 0,45 µm puis on coule 25 ml de méthanol à une température voisine de 10°C. La solution est ensuite amenée à 20°C et agité 15 minutes. On ajoute alors 63 ml de méthanol puis on agite 30 minutes. L'agitation est alors arrêtée et on laisse sédimenter la suspension pendant 30 minutes. Le surnageant est ensuite prélevé puis écarté (70 ml). Sur le précipité sédimenté, on ajoute 70 ml de méthanol et on agite pendant 30 minutes. On laisse re-sédimenter le précipité quelques minutes. La suspension est ensuite filtrée sur verre fritté 3. Le gâteau blanc obtenu est ensuite lavé par 2 fois 30 ml de méthanol. Le solide humide est essoré puis séché sous pression réduite, à une température voisine de 60°C. Après séchage, on obtient 2,5 g d'héparine dépolymérisée pure (sel de sodium). Le rendement obtenu est de 71,4 %.

Les caractéristiques de l'héparine dépolymérisée ainsi obtenue sont les suivantes :
Poids moléculaire moyen : 2600 Daltons
Activité anti-Xa : 150,5 UI/mg
Activité anti-IIa : 3,2 UI/mg
Rapport activité anti-Xa/activité anti-IIa : 47

### EXEMPLE 10

### Dépolymérisation et transformation en sel de sodium (0,8 équivalents de base):

Dans un erlenmeyer A, on introduit 70 ml de dichlorométhane. Sous agitation, on charge lentement 10 g (0,006 mole) d'ester benzylique d'héparine (taux d'estérification : 75 %, sel de benzéthonium) obtenu comme décrit dans l'exemple A. Après dissolution complète à une température voisine de 30°C et retour à une température voisine de 20°C, on ajoute 1,38 ml (0,004 mole) de 2-tert-butylimino-2-diethylamino-1,3-diméthylperhydro-1,2,3-diazaphosphorine. On agite à une température voisine de 20°C, pendant 24 heures. Pendant ce temps, on prépare dans un erlenmeyer B, une solution de 30 g d'acétate de sodium anhydre dans 300 ml de méthanol. Sous agitation magnétique, le mélange réactionnel de l'erlenmeyer A est coulé en 1 minute 15 secondes dans la solution méthanolique d'acétate de sodium à une température voisine de 4°C. Après 5 minutes d'agitation, on laisse décanter la suspension pendant 1 heure. La partie limpide du surnageant est prélevée et écartée (190 ml). Sur le précipité sédimenté, on ajoute 190 ml de méthanol et on agite pendant 5 minutes. On laisse re-sédimenter le précipité 30 minutes environ. La partie limpide du surnageant est prélevée et écartée (190 ml). Sur le précipité sédimenté, on ajoute 190 ml de méthanol et on agite pendant 30 minutes. Le surnageant est prélevé et écarté (190 ml). Le précipité en suspension est ensuite filtré sur verre fritté 3. Le gâteau obtenu est ensuite lavé par 150 ml de méthanol. Le solide humide jaune clair est essoré puis séché sous pression réduite (6 kPa), à une température voisine de 40°C. Après 18 heures de séchage, on obtient 3,05 g d'héparine dépolymérisée brute (sel de sodium). Le rendement obtenu est de 80 %.

### Saponification :

Dans un erlenmeyer de 100 ml, on introduit 3,05 g (0,0048 mole) de l'héparine dépolymérisée brute (sel de sodium) obtenue précédemment et 21 ml d'eau. La solution est filtrée sur verre fritté 3 et rincé par 2 fois 6 ml d'eau. Le filtrat obtenu est chargé dans un erlenmeyer de 150 ml. Sous agitation magnétique, on introduit 0,6 ml (0,006 mole) de lessive de soude à 30 %, à une température voisine de 4°C. Après addition, le mélange est agité pendant 2 heures. On neutralise la solution par ajout d'HCl 1N et on ajoute 4 g de chlorure de sodium. On ajoute au milieu réactionnel 28 ml de méthanol. Après 15 minutes d'agitation on ajoute 72 ml de méthanol puis on agite pendant 1 heure. L'agitation est alors arrêtée et on laisse sédimenter la suspension pendant 30 minutes à une température voisine de 4°C. Le surnageant est ensuite prélevé puis écarté (80 ml). Sur le précipité sédimenté, on ajoute 80 ml de méthanol et on agite pendant 5 minutes. On laisse re-sédimenter le précipité 30 minutes environ. Le surnageant est prélevé et écarté (80 ml). Sur le précipité sédimenté, on ajoute 80 ml de méthanol et on agite pendant 30 minutes. Le précipité en suspension est ensuite filtré sur verre fritté 3. Le gâteau obtenu est ensuite lavé par 50 ml de méthanol. Le solide humide est essoré puis séché sous pression réduite (6 kPa), à une température voisine de 40°C. Après séchage, on obtient 1,6 g d'héparine dépolymérisée brute (sel de sodium). Le rendement obtenu est de 57 %.

### Purification :

Dans un erlenmeyer de 50 ml, on introduit 1,6 g d'héparine dépolymérisée brute obtenue précédemment et 16 ml d'eau distillée. Le mélange est porté à 40°C sous agitation magnétique. Par addition de soude 1N, on porte le pH à 9,6 ± 0,1. Le milieu réactionnel est filtré sur membrane 0,45 µm et on ajoute 0,08 ml d'une solution aqueuse de peroxyde d'hydrogène à 30 %. Après environ 2 heures d'agitation, à une température voisine de 20°C le mélange est neutralisé par ajout d'HCl à 1 N, puis on ajoute 2 g de chlorure de sodium. La solution est ensuite filtrée sur membrane 0,45 µm puis on coule 14 ml de méthanol à une température voisine de 10°C. La solution est ensuite amenée à 20°C et agité 15 minutes. On ajoute alors 36 ml de méthanol puis on agite 1 heure. L'agitation est alors arrêtée et on laisse sédimenter la suspension pendant 30 minutes. Le surnageant est ensuite prélevé puis écarté (50 ml). Sur le précipité sédimenté, on ajoute 50 ml de méthanol et on agite pendant 5 minutes. On laisse re-sédimenter le précipité 30 minutes environ. Le surnageant est prélevé et écarté (50 ml). Le précipité en suspension est ensuite filtré sur verre fritté 3. Le solide humide est essoré puis séché sous pression réduite (6 kPa), à une température voisine de 40°C. Après séchage, on obtient 1,25 g d'héparine dépolymérisée pure (sel de sodium). Le rendement obtenu est de 78 %.

Les caractéristiques de l'héparine dépolymérisée ainsi obtenue sont les suivantes :
Poids moléculaire moyen : 2400 Daltons
Activité anti-Xa : 154,3 UI/mg
Activité anti-IIa : 1,6 UI/mg
Rapport activité anti-Xa/activité anti-IIa : 96,4

### EXEMPLE 11

### Dépolymérisation et transformation en sel de sodium :

Dans un erlenmeyer A, on introduit 140 ml de dichlorométhane. Sous agitation, on charge lentement 20 g (0,019 mole) d'ester benzylique d'héparine (taux d'estérification : 75 %, sel de benzéthonium) obtenu comme décrit dans l'exemple A. Après dissolution complète à une température voisine de 40°C et retour à une température voisine de 20°C, on ajoute 3,5 ml (0,011 mole) de tert-butylimino-tri(pyrrolidino)phosphorane. On agite à une température voisine de 20°C, pendant 24 heures. Pendant ce temps, on prépare dans un erlenmeyer B, une solution de 60 g d'acétate de sodium anhydre dans 600 ml de méthanol. Après dissolution complète on ajoute à la solution 10 g de célite Hyflo supercel. Sous agitation magnétique, le mélange réactionnel de l'erlenmeyer A est coulé en 1 minute 30 secondes dans la solution méthanolique d'acétate de sodium à une température voisine de 4°C. Après 5 minutes d'agitation on laisse décanter la suspension pendant 30 minutes. La partie limpide du surnageant est prélevée et écartée (400 ml). Sur le précipité sédimenté, on ajoute 400 ml de méthanol et on agite pendant 5 minutes. On laisse re-sédimenter le précipité 1 heure 20 minutes environ. Le surnageant est prélevé et écarté (250 ml). Sur le précipité sédimenté, on ajoute 250 ml de méthanol et on agite pendant 5 minutes. Le précipité en suspension est ensuite filtré sur verre fritté 3. Le gâteau obtenu est ensuite lavé par 200 ml de méthanol. Le solide humide jaune clair est essoré puis séché sous pression réduite (6 kPa), à une température voisine de 40°C. Après séchage, on obtient 5,39 g d'héparine dépolymérisée (ester de benzyle, sel de sodium). Le rendement obtenu est de 69 %.

### Saponification :

Dans un erlenmeyer de 50 ml, on introduit 5 g (0,0076 mole) de l'héparine dépolymérisée (ester de benzyle, sel de sodium) obtenue précédemment et 35 ml d'eau. La solution est filtrée sur verre fritté 3 et rincé par 2 fois 10 ml d'eau. Le filtrat obtenu est chargé dans un erlenmeyer de 250 ml. Sous agitation magnétique, on introduit 1 ml (0,01 mole) de lessive de soude à 30 %, à une température voisine de 4°C. Après addition, le mélange est agité pendant 2 heures. On neutralise la solution par ajout d'HCl 1N et on ajoute 6 g de chlorure de sodium. On ajoute au milieu réactionnel 42 ml de méthanol. Après 15 minutes d'agitation on ajoute 104 ml de méthanol puis on agite pendant 15 minutes. L'agitation est alors arrêtée et on laisse sédimenter la suspension pendant 1 heure à une température voisine de 4°C. Le surnageant est ensuite prélevé puis écarté (140 ml). Sur le précipité sédimenté, on ajoute 140 ml de méthanol et on agite pendant 5 minutes. On laisse re-sédimenter le précipité 45 minutes environ. Le surnageant est prélevé et écarté (160 ml). Sur le précipité sédimenté, on ajoute 160 ml de méthanol et on agite pendant 5 minutes. Le précipité en suspension est ensuite filtré sur verre fritté 3. Le gâteau obtenu est ensuite lavé par 100 ml de méthanol. Le solide humide est essoré puis séché sous pression réduite (6 kPa), à une température voisine de 40°C. Après 48 heures de séchage, on obtient 2,7 g d'héparine dépolymérisée brute (sel de sodium). Le rendement obtenu est de 59 %.

### Purification :

Dans un erlenmeyer de 50 ml, on introduit 2,6 g d'héparine dépolymérisée brute obtenue précédemment et 25 ml d'eau distillée. Le mélange est porté à 40°C sous agitation magnétique. Par addition de soude 1N, on porte le pH à 9,7 ± 0,1. Le milieu réactionnel est filtré sur membrane 0,45 µm et on ajoute 0,15 ml d'une solution aqueuse de peroxyde d'hydrogène à 30 %. Après environ 2 heures d'agitation, à une température voisine de 20°C le mélange est neutralisé par ajout d'HCl 1N, puis on ajoute 3 g de chlorure de sodium. La solution est ensuite filtrée sur membrane 0,45µm puis on coule 21 ml de méthanol à une température voisine de 10°C. La solution est ensuite amenée à 20°C et agité 15 minutes. On ajoute alors 54 ml de méthanol puis on agite une heure. L'agitation est alors arrêtée et on laisse sédimenter la suspension pendant 20 minutes. Le surnageant est ensuite prélevé puis écarté (50 ml). Sur le précipité sédimenté, on ajoute 50 ml de méthanol et on agite pendant 5 minutes. On laisse re-sédimenter le précipité 20 minutes environ. Le surnageant est prélevé et écarté (50 ml). Le précipité en suspension est ensuite filtré sur verre fritté 3. Le gâteau blanc obtenu est ensuite lavé par 50 ml de méthanol. Le solide humide est essoré puis séché sous pression réduite (6 kPa), à une température voisine de 40°C. Après 18 heures de séchage, on obtient 2,35 g d'héparine dépolymérisée pure (sel de sodium). Le rendement obtenu est de 90 %.

Les caractéristiques de l'héparine dépolymérisée ainsi obtenue sont les suivantes :
Poids moléculaire moyen : 2400 Daltons
Activité anti-Xa : 167,5 UI/mg
Activité anti-IIa : 1,1 UI/mg
Rapport activité anti-Xa/activité anti-IIa : 152

### EXEMPLE 12

### Dépolymérisation et transformation en sel de sodium (0, 05 % d'eau) :

Dans un erlenmeyer, on introduit 140 ml de dichlorométhane. Sous agitation, on charge lentement 20 g (0,019 mole) d'ester benzylique d'héparine (taux d'estérification : 75%, sel de benzéthonium) obtenu comme décrit dans l'exemple A. On ajoute 20 g de tamis moléculaires 4 A au milieu réactionnel et la teneur en eau est amenée à 0,05% en agitant lentement pendant 48h . Le surnageant est transféré sous atmosphère inerte dans un erlenmeyer A. Après dissolution complète, on ajoute 3,5 ml (0,012 mole) de 2-tert-butylimino-2-diethylamino-1 ,3-diméthylperhydro-1,2,3-diaza-phosphorine. On agite à une température voisine de 20°C, pendant 24 heures. Pendant ce temps, on prépare dans un erlenmeyer B, une solution de 60 g d'acétate de sodium anhydre dans 600 ml de méthanol. Après dissolution complète, on ajoute à la solution 10 g de célite Hyflo supercel. Sous agitation magnétique, le mélange réactionnel de l'erlenmeyer A est coulé en environ 2 minutes dans la solution méthanolique d'acétate de sodium à une température voisine de 4°C. Après 15 minutes d'agitation on laisse décanter la suspension pendant 1 heure. La partie limpide du surnageant est prélevée et écartée (420 ml). Sur le précipité sédimenté, on ajoute 420 ml de méthanol et on agite pendant 15 minutes. On laisse re-sédimenter le précipité 1 heure environ. Le surnageant est prélevé et écarté (450 ml). Sur le précipité sédimenté, on ajoute 450 ml de méthanol et on agite pendant 15 minutes. La suspension est ensuite filtré sur verre fritté 3. Le gâteau obtenu est ensuite lavé par 200 ml de méthanol. Le solide humide jaune clair est essoré puis séché sous pression réduite (6 kPa), à une température voisine de 50°C. Après 16 heures de séchage, on obtient 5,36 g d'héparine dépolymérisée brute, sel de sodium dans la célite (10 g). Le rendement obtenu est de 68,6 %.

### Saponification :

Dans un erlenmeyer de 50 ml, on introduit 5,36 g (0,00817 mole) de l'héparine dépolymérisée brute, sel de sodium dans la célite (10 g) obtenue précédemment et 50 ml d'eau. La suspension est filtrée sur verre fritté 3 et rincé par 4 fois 15 ml d'eau. Le filtrat obtenu est chargé dans un erlenmeyer de 150 ml. Sous agitation magnétique, on introduit 1,01 ml (0,0122 mole) de lessive de soude à 35%, à une température voisine de 4°C. Après addition, le mélange est agité pendant 3 heures. On neutralise la solution par ajout d'HCl 1N et on ajoute 11 g de chlorure de sodium. On ajoute au milieu réactionnel 77 ml de méthanol. Après 15 minutes d'agitation, on ajoute 200 ml de méthanol puis on agite pendant une heure. L'agitation est alors arrêtée et on laisse sédimenter la suspension pendant 1 heure à une température voisine de 4°C. Le surnageant est ensuite prélevé puis écarté (240 ml). Sur le précipité sédimenté, on ajoute 240 ml de méthanol et on agite pendant 10 minutes. On laisse re-sédimenter le précipité 30 minutes environ. Le surnageant est prélevé et écarté (225 ml). Sur le précipité sédimenté, on ajoute 225 ml de méthanol et on agite pendant 10 minutes. La suspension est ensuite filtré sur verre fritté 3. Le gâteau obtenu est ensuite lavé par 2 fois 50 ml de méthanol. Le solide humide est essoré puis séché sous pression réduite (6 kPa), à une température voisine de 40°C. Après 18 heures de séchage, on obtient 2,65 g d'héparine dépolymérisée brute (sel de sodium). Le rendement obtenu est de 53,7 %.

### Purification :

Dans un erlenmeyer de 100 ml, on introduit 2,65 g d'héparine dépolymérisée brute obtenue précédemment et 26,5 ml d'eau distillée. Le mélange est porté à 40°C sous agitation magnétique. Par addition de soude 1N, on porte le pH à 9,7 ± 0.1. Le milieu réactionnel est filtré sur membrane 0,45 µm et on ajoute 0,25 ml d'une solution aqueuse de peroxyde d'hydrogène à 30%. Après environ 2 heures d'agitation, à une température voisine de 20°C le mélange est neutralisé par ajout d'HCl à 1 N, puis on ajoute 3 g de chlorure de sodium. La solution est ensuite filtrée sur membrane 0.45µm puis on coule 21 ml de méthanol à une température voisine de 10°C. La solution est ensuite amenée à 20°C et agité 15 minutes. On ajoute alors 54 ml de méthanol puis on agite une heure. L'agitation est alors arrêtée et on laisse sédimenter la suspension pendant 45 minutes. Le surnageant est ensuite prélevé puis écarté (46 ml). Sur le précipité sédimenté, on ajoute 46 ml de méthanol et on agite pendant 5 minutes. On laisse re-sédimenter le précipité 30 minutes environ. Le surnageant est prélevé et écarté (50 ml). 50 ml de méthanol sont ajoutés et le précipité en suspension est ensuite filtré sur verre fritté 4. Le gâteau blanc obtenu est ensuite lavé par 2 portions de 10 ml de méthanol. Le solide humide est essoré puis séché sous pression réduite (6 kPa), à une température voisine de 50°C. Après 18 heures de séchage, on obtient 2,363 g d'héparine dépolymérisée pure (sel de sodium). Le rendement obtenu est de 89,1%.

Les caractéristiques de l'héparine dépolymérisée ainsi obtenue sont les suivantes :
Poids moléculaire moyen : 2500 Daltons
Activité anti-Xa : 192 UI/mg
Activité anti-IIa : 1,3 UI/mg
Rapport activité anti-Xa/activité anti-IIa : 148

## Revendications

1. Mélanges d'oligosaccharides sulfatés possédant la structure générale des polysaccharides constitutifs de l'héparine et présentant les caractéristiques suivantes :
- ils ont un poids moléculaire moyen de 2000 à 3000 daltons,
- ils ont une activité anti-Xa > 150 UI/mg et < 200 UI/mg,
- ils ont une activité anti-IIa inférieure à 10 UI/mg,
- ils ont un rapport activité anti-Xa/activité anti-IIa supérieur à 30,
- les oligosaccharides constitutifs des mélanges :
- contiennent 2 à 26 motifs saccharidiques,
- ont un motif acide uronique-4,5 insaturé 2-O-sulfate à l'une de leurs extrémités,
- contiennent une fraction hexasaccharide représentant de 15 à 25 % des mélanges d'oligosaccharides,
- contiennent de 8 à 15 % de l'hexasaccharide ΔIIa-IIs-Is dans la fraction hexasaccharide 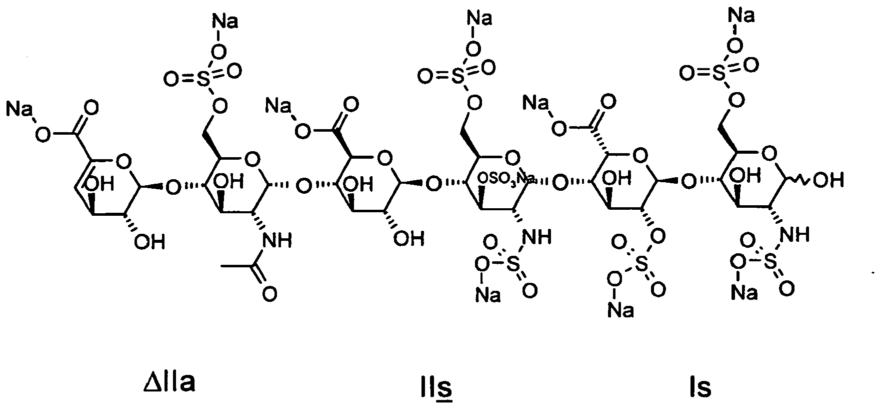 et sont sous forme d'un sel de métal alcalin ou alcalinoterreux.

2. Mélange d'oligosaccharides selon la revendication 1 **caractérisé en ce que** le sel de métal alcalin ou alcalinoterreux est choisi parmi les sels de sodium, potassium, calcium et magnésium.

3. Mélange d'oligosaccharides selon l'une quelconque des revendications 1 à 2 **caractérisé en ce qu'**il a activité anti-IIa inférieure à 5 UI/mg .

4. Mélanges d'oligosaccharides selon l'une quelconque des revendications 1 à 3 **caractérisé en ce qu'**il présente un rapport activité anti-Xa/activité anti-IIa supérieur à 50 .

5. Mélanges d'oligosaccharides selon l'une quelconque des revendications 1 à 4 **caractérisé en ce qu'**il a un poids moléculaire moyen compris entre 2400 et 2650 Da.

6. Mélange d'oligosaccharides selon l'une quelconque des revendications 1 à 5 **caractérisé en ce qu'**il présente une activité anti-Xa > 150 UI/mg et < 200 UI/mg, une activité anti-IIa comprise entre 0,5 et 3,5 UI/mg, et un poids moléculaire moyen compris entre 2400 et 2650 Da.

7. Procédé de préparation des mélanges d'oligosaccharides tels que définis selon l'une quelconque des revendications 1 à 6, au cours duquel on dépolymérise le sel d'ammonium quaternaire de l'ester benzylique de l'héparine en milieu organique en présence d'une base organique forte de pKa supérieur à 20, **caractérisé en ce que** la base organique utilisée consiste en la famille des phosphazènes renfermant un pourcentage d'eau inférieur à 0,3 %.

8. Procédé de préparation selon la revendication 7 de mélanges d'oligosaccharides selon la revendication 1, **caractérisé en ce que** la teneur en eau est inférieure à 0,2 %.

9. Procédé de préparation selon les revendications 7 ou 8 **caractérisé en ce que** le sel d'ammonium quaternaire de l'ester benzylique de l'héparine est le sel de benzéthonium, de cétylpyridinium ou de cétyltriméthyl ammonium.

10. Procédé de préparation selon les revendications 7 ou 8 **caractérisé en ce que** les bases de la famille des phosphazènes sont celles de formule : 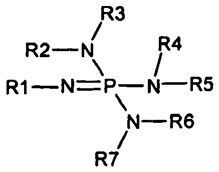 dans laquelle les radicaux R₁ à R₇ sont identiques ou différents et représentent des radicaux alkyle linéaire ou ramifié renfermant de 1 à 6 atomes de carbone.

11. Procédé de préparation selon la revendication 10 **caractérisé en ce que** la base de la famille des phosphazènes utilisée lors de l'étape de dépolymérisation est le 2-tert-butylimino-2-diethylamino-1,3-diméthylperhydro-1,2,3-diazaphosphorine ou le tert-butylimino-tri(pyrrolidino)-phosphorane.

12. Procédé de préparation selon les revendications 7 ou 8 **caractérisé en ce que** le rapport en mole base forte/ester est compris entre 0,2 et 5.

13. Procédé de préparation selon les revendications 7 ou 8 dans lequel on effectue les opérations suivantes :
a) Transalification de l'héparine de sodium par action de chlorure de benzéthonium,
b) Estérification de l'héparinate de benzéthonium obtenu par action de chlorure de benzyle,
c) Transalification de l'ester benzylique obtenu et obtention du sel d'ammonium quaternaire,
d) Dépolymérisation du sel d'ammonium quaternaire de l'ester benzylique de l'héparine par la méthode telle que définie à la revendication 7 ou 8,
e) Transformation du sel d'ammonium quaternaire en sel de sodium,
f) Eventuellement saponification de l'héparine par action d'une base,
g) Eventuellement purification

14. Procédé selon la revendication 13 **caractérisé en ce que** la réaction de l'étape a) est réalisée par action du chlorure de benzéthonium en excès, sur l'héparine sodique, à une température voisine de 15 à 25°C avec un rapport molaire sel/héparine sodique compris entre 3 et 4.

15. Procédé selon la revendication 13 **caractérisé en ce que** l'estérification de l'étape b) s'effectue au sein d'un solvant organique chloré à une température comprise entre 25 et 45°C, et l'ester sous forme de sel de sodium est ensuite récupéré par précipitation au moyen d'acétate de sodium à 10 % en poids dans un alcool à raison d'1 à 1,2 volume d'alcool par volume de milieu réactionnel.

16. Procédé selon la revendication 13 ou 15 **caractérisé en ce que** le taux d'estérification du sel d'ammonium quaternaire de l'ester benzylique de l'héparine est compris entre 50 et 100 %.

17. Procédé selon la revendication 13, 15 ou 16 **caractérisé en ce qu'**on utilise de 0,5 à 1,5 parties en poids du chlorure de benzyle pour 1 partie en poids du sel de benzéthonium de l'héparine avec un temps de réaction qui sera compris entre 10 et 35 h.

18. Procédé selon la revendication 13 **caractérisé en ce que** la transalification de l'étape c) s'effectue au moyen d'un chlorure d'ammonium quaternaire en milieu aqueux, à une température comprise entre 10 et 25°C.

19. Procédé selon la revendication 18 **caractérisé en ce que** le rapport en mole chlorure d'ammonium quaternaire/sel de sodium de l'ester benzylique de l'héparine est compris entre 2 et 3.

20. Procédé selon la revendication 13 **caractérisé en ce que** la transformation en sel de sodium du sel d'ammonium quaternaire de l'ester benzylique de l'héparine dépolymérisée (étape e) est effectuée par traitement du milieu réactionnel avec une solution alcoolique d'acétate de sodium , à une température comprise entre 15 et 25°C.

21. Procédé selon la revendication 13 **caractérisé en ce que** la saponification (étape f) s'effectue au moyen d'un hydroxyde de métal alcalin en milieu aqueux, à une température comprise entre 0 et 20°C.

22. Procédé selon la revendication 21 **caractérisé en ce qu'**on utilise de 1 à 5 équivalents molaires d'hydroxyde de métal alcalin .

23. Procédé selon la revendication 13 **caractérisé en ce qu'**on purifie (étape g) au moyen de peroxyde d'hydrogène, en milieu aqueux, à une température de 10 à 50°C .

24. Médicament contenant les mélanges d'oligosaccharides selon l'une quelconque des revendications 1 à 6.

25. Médicament selon la revendication 24, **caractérisé en ce qu'**il présente une activité antithrombotique.

26. Médicaments selon les revendications 24 ou 25 pour la prévention ou le traitement des thromboses veineuses et artérielles, de la thrombose veineuse profonde, de l'embolie pulmonaire, de l'angor instable, de l'infarctus du myocarde, de l'ischémie cardiaque, des maladies occlusives des artères périphériques, de la fibrillation auriculaire, de la prolifération des cellules musculaires lisses, de l'athérosclérose et l'artériosclérose, du cancer par la modulation de l'angiogénèse et des facteurs de croissance, et des désordres diabétiques.

27. Compositions pharmaceutiques renfermant au moins un médicament tel que défini à la revendication 24 et un ou plusieurs excipients ou véhicules ou additifs pharmaceutiquement inertes.

28. Compositions pharmaceutiques selon la revendication 27 **caractérisées en ce qu'**elles consistent en solutions injectables par voie sous-cutanée ou intraveineuse.

29. Compositions pharmaceutiques selon la revendication 27 **caractérisées en ce qu'**elles consistent en une formulation par inhalation destinée à la voie pulmonaire.

30. Compositions pharmaceutiques selon la revendication 27 **caractérisées en ce qu'**elles consistent en une formulation pour une administration destinée à la voie orale.

31. Mélanges d'oligosaccharides sulfatés selon la revendication 1, susceptibles d'être obtenus par le procédé tel que défini à la revendication 13.

## Claims

1. Mixtures of sulfated oligosaccharides having the general structure of the constituent polysaccharides of heparin and having the following characteristics:
- they have a mean molecular weight of 2000 to 3000 daltons,
- they have an anti-Xa activity > 150 IU/mg and < 200 IU/mg,
- they have an anti-IIa activity of less than 10 IU/mg,
- they have an anti-Xa activity/anti-IIa activity ratio of greater than 30,
- the constituent oligosaccharides of the mixtures:
- contain 2 to 26 saccharide units,
- have a 4,5-unsaturated uronic acid 2-O-sulfate unit at one of their ends,
- contain a hexasaccharide fraction representing from 15 to 25% of the mixtures of oligosaccharides,
- contain from 8 to 15% of the hexasaccharide ΔIIa-IIs-Is in the hexasaccharide fraction 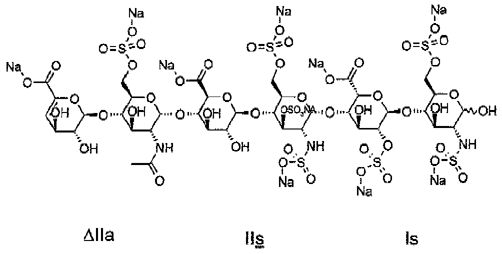 and are in the form of an alkali or alkaline-earth metal salt.

2. Mixture of oligosaccharides according to Claim 1, **characterized in that** the alkali or alkaline-earth metal salt is chosen from sodium, potassium, calcium and magnesium salts.

3. Mixture of oligosaccharides according to either of Claims 1 and 2, **characterized in that** it has an anti-IIa activity of less than 5 IU/mg.

4. Mixtures of oligosaccharides according to any one of Claims 1 to 3, **characterized in that** it exhibits an anti-Xa activity/anti-IIa activity ratio greater than 50.

5. Mixtures of oligosaccharides according to any one of Claims 1 to 4, **characterized in that** it has a mean molecular weight of between 2400 and 2650 Da.

6. Mixture of oligosaccharides according to any one of Claims 1 to 5, **characterized in that** it exhibits an anti-Xa activity > 150 IU/mg and < 200 IU/mg, an anti-IIa activity of between 0.5 and 3.5 IU/mg, and a mean molecular weight of between 2400 and 2650 Da.

7. Method for preparing mixtures of oligosaccharides as defined according to any one of Claims 1 to 6, during which the quaternary ammonium salt of the benzyl ester of heparin is depolymerized in an organic medium in the presence of a strong organic base with a pKa greater than 20, charactarized in that the organic base used consists of the family of phosphazenes, containing a percentage of water of less than 0.3%.

8. Method according to Claim 7 for preparing mixtures of oligosaccharides according to Claim 1, **characterized in that** the water content is less than 0.2%.

9. Method of preparation according to Claims 7 and 8, **characterized in that** the quaternary ammonium salt of the benzyl ester of heparin is the benzethonium, cetylpyridinium or cetyltrimethylammonium salt.

10. Method of preparation according to Claims 7 and 8, **characterized in that** the bases of the family of phosphazenes are those of formula: 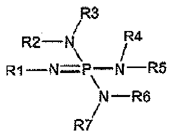 in which the radicals R₁ to R₇ are identical or different and represent linear or branched alkyl radicals containing from 1 to 6 carbon atoms.

11. Method of preparation according to Claim 10, **characterized in that** the base of the family of phosphazenes used during the depolymerization step is 2-tert-butylimino-2-diethylamino-1,3-dimethylperhydro-1,2,3-diazaphosphorine or tert-butylimino-tri(pyrroli-dino)phosphorane.

12. Method of preparation according to Claims 7 and 8, **characterized in that** the strong base/ester mol ratio is between 0.2 and 5.

13. Method of preparation according to Claims 7 and 8, in which the following operations are carried out:
a) transalification of sodium heparin by the action of benzethonium chloride,
b) esterification of benzethonium heparinate obtained by the action of benzyl chloride,
c) transalification of the benzyl ester obtained and obtaining of the quaternary ammonium salt,
d) depolymerization of the quaternary ammonium salt of the benzyl ester of heparin by the method as defined in Claim 7 or 8,
e) conversion of the quaternary ammonium salt to a sodium salt,
f) optionally saponification of the heparin by the action of a base,
g) optionally purification.

14. Method according to Claim 13, **characterized in that** the reaction of step a) is carried out by the action of benzethonium chloride in excess, on sodium heparin, at a temperature in the region of 15 to 25°C, with a salt/ sodium heparin molar ratio of between 3 and 4.

15. Method according to Claim 13, **characterized in that** the esterification of step b) is carried out in a chlorinated organic solvent, at a temperature of between 25 and 45°C, and the ester in the form of a sodium salt is then recovered by precipitation by means of sodium acetate at 10% by weight in an alcohol in a proportion of 1 to 1.2 volumes of alcohol per volume of reaction medium.

16. Method according to Claim 13 or 15, **characterized in that** the degree of esterification of the quaternary ammonium salt of the benzyl ester of heparin is between 50 and 100%.

17. Method according to Claim 13, 15 or 16, **characterized in that** 0.5 to 1.5 parts by weight of benzyl chloride per 1 part by weight of benzethonium salt of heparin are used with a reaction time which will be between 10 and 35 h.

18. Method according to Claim 13, **characterized in that** the transalification of step c) is carried out by means of a quaternary ammonium chloride, in an aqueous medium at a temperature between 10 and 25°C.

19. Method according to Claim 18, **characterized in that** the quaternary ammonium chloride/sodium salt of the benzyl ester of heparin mol ratio is between 2 and 3.

20. Method according to Claim 13, **characterized in that** the conversion to a sodium salt of the quaternary ammonium salt of the benzyl ester of depolymerized heparin (step e) is carried out by treating the reaction medium with an alcoholic solution of sodium acetate, at a temperature between 15 and 25°C.

21. Method according to Claim 13, **characterized in that** the saponification (step f) is carried out by means of an alkali metal hydroxide, in an aqueous medium, at a temperature between 0 and 20°C.

22. Method according to Claim 21, **characterized in that** 1 to 5 molar equivalents of alkali metal hydroxide are used.

23. Method according to Claim 13, **characterized in that** the purification (step g) is carried out by means of hydrogen peroxide, in an aqueous medium, at a temperature of 10 to 50°C.

24. Medicament containing the mixtures of oligosaccharides according to any one of Claims 1 to 6.

25. Medicament according to Claim 24, **characterized in that** it exhibits an antithrombotic activity.

26. Medicaments according to Claims 24 and 25, for the prevention or treatment of venous and arterial thromboses, deep vein thrombosis, pulmonary embolism, unstable angina, myocardial infarction, cardiac ischemia, occlusive diseases of the peripheral arteries and atrial fibrillation, the proliferation of smooth muscle cells, atheriosclerosis and arteriosclerosis, cancer by modulating angiogenesis and growth factors, and diabetic disorders.

27. Pharmaceutical compositions containing at least one medicament as defined in Claim 24 and one or more pharmaceutically inert excipients or vehicles or additives.

28. Pharmaceutical compositions according to Claim 27, **characterized in that** they consist of solutions for injection by the subcutaneous or intravenous route.

29. Pharmaceutical compositions according to Claim 27, **characterized in that** they consist of a formulation for inhalation intended for the pulmonary route.

30. Pharmaceutical compositions according to Claim 27, **characterized in that** they consist of a formulation for administration intended for the oral route.

31. Mixtures of sulphated oligosaccharides according to Claim 1, which can be obtained by the method as defined in Claim 13.

## Patentansprüche

1. Gemische von sulfatisierten Oligosaachariden, die die allgemeine Struktur der Polysaccharide besitzen, die Heparin bilden, und die folgenden Eigenschaften aufweisen:
- sie haben eine mittlere Molekülmasse von 2000 bis 3000 Dalton,
- sie haben eine Anti-Xa-Aktivität > 150 IU/mg und < 200 IU/mg,
- sie haben eine Anti-IIa-Aktivität von weniger als 10 IU/mg,
- sie haben ein Verhältnis der Anti-Xa-/Anti-IIa-Aktivität von mehr als 30,
- die Oligosaccharide, die die Gemische bilden:
- enthalten 2 bis 26 Saccharidmotive,
- weisen ein 4,5-ungesättigte-Uronsäure-2-O-sulfat-Motiv an einem ihrer Enden auf,
- enthalten eine Hexasaccharidfraktion, die 15 bis 25% der Gemische von oligosacchariden ausmacht,
- enthalten 8 bis 15% des Hexasaccharids ΔIIa-IIs-Is in der Hexasaccharidfraktion 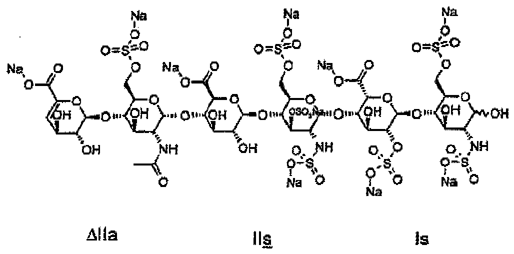 und liegen in Form eines Alkalimetall- oder Erdalkalimetallsalzes vor.

2. Gemisch von oligosacchariden nach Anspruch 1, **dadurch gekennzeichnet, dass** das Alkallmetall- oder Erdalkalimetallsalz aus Natrium-, Kalium-, Calcium- und Magnesiumsalzen ausgewählt ist.

3. Gemisch von oligosacchariden nach einem der Ansprüche 1 bis 2, **dadurch gekennzeichnet, dass** es eine Anti-IIa-Aktivität von weniger als 5 IU/mg hat.

4. Gemische von Oligosacchariden nach einem der Ansprüche 1 bis 3, **dadurch gekennzeichnet, dass** es ein Verhältnis der Anti-Xa-/Anti-IIa-Aktivität von mehr als 50 aufweist.

5. Gemische von Oligosacchariden nach einem der Ansprüche 1 bis 4, **dadurch gekennzeichnet, dass** es eine mittlere Molekülmasse zwischen 2400 und 2650 Dalton hat.

6. Gemisch von Oligosacchariden nach einem der Ansprüche 1 bis 5, **dadurch gekennzeichnet, dass** es eine Anti-Xa-Aktivität > 150 IU/mg und 200 IU/mg, eine Anti-IIa-Aktivität zwischen 0,5 und 3,5 IU/mg und eine mittlere Molekülmasse zwischen 2400 und 2650 Dalton aufweist.

7. Verfahren zur Herstellung von Gemischen von Oligosacchariden nach einem der Ansprüche 1 bis 6, bei dem man das quaternäre ammoniumsalz des Benzylesters von Heparin in organischem Medium in Gegenwart einer starken organischen Base mit einer pKa über 20 depolymerisiert, **dadurch gekennzeichnet, dass** die verwendete organische Base aus der Familie der Phosphazene besteht, wobei weniger als 0,3% Wasser enthalten ist.

8. Verfahren zur Herstellung nach Anspruch 7 von Gemischen von Oligosacchariden nach Anspruch 1, **dadurch gekennzeichnet, dass** der Gehalt an Wasser kleiner als 0,2% ist.

9. Verfahren zur Herstellung nach den Ansprüchen 7 oder 8, **dadurch gekennzeichnet, dass** das quaternäre Ammoniumsalz des Benzylesters von Heparin das Benzethonium-, Cetylpyridinium- oder Cetyltrimethylammoniumsalz ist.

10. Verfahren zur Herstellung nach den Ansprüchen 7 oder 8, **dadurch gekennzeichnet, dass** die Basen aus der Familie der Phosphazene diejenigen der folgenden Formel sind: 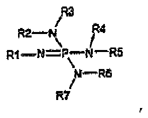 worin die Reste R₁ bis R₇ gleich oder verschieden sind und für gerade oder verzweigte Alkylreste mit 1 bis 6 Kohlenstoffatomen stehen.

11. Verfahren zur Herstellung nach Anspruch 10, **dadurch gekennzeichnet, dass** die im Depolymerisationsschritt verwendete Base aus der Familie der Phosphazene 2-tert.-Butylimino-2-diethylamino-1,3-dimethylperhydro-1,2,3-diazaphosphorin oder tert.-Butylimino-tri(pyrrolidino)phosphoran ist.

12. Verfahren zur Herstellung nach den Ansprüchen 7 oder 8, **dadurch gekennzeichnet, dass** das Molverhältnis starke Base/Ester zwischen 0,2 und 5 beträgt.

13. Verfahren zur Herstellung nach den Ansprüchen 7 oder 8, **dadurch gekennzeichnet, dass** man die folgenden Arbeitsschritte durchführt:
a) Umsalzung von Natrium-Heparin durch Einwirkung von Benzethoniumchlorid,
b) Veresterung des erhaltenen Benzethoniumheparinats durch Einwirkung von Benzylchlorid,
c) Umsalzung des erhaltenen Benzylesters und Gewinnen des quaternären Ammoniumsalzes,
d) Depolymerisation des quaternären Ammoniumsalzes des Benzylesters von Heparin durch das Verfahren nach Anspruch 7 oder 8,
e) Umwandeln des quaternären Ammoniumsalzes in das Natriumsalz,
f) schließlich Verseifung des Heparins durch Einwirkung einer Base,
g) schließlich Reinigung.

14. Verfahren nach Anspruch 13, **dadurch gekennzeichnet, dass** die Reaktion von Schritt a) durch Einwirkung von Benzethoniumchlorid im Überschuss auf Natrium-Heparin bei einer Temperatur von etwa 15 bis 25°C mit einem Molverhältnis von Salz/Natrium-Heparin zwischen 3 und 4 durchgeführt wird.

15. Verfahren nach Anspruch 13, **dadurch gekennzeichnet, dass** man die Veresterung in Schritt b) in einem chlorierten organischen Lösungsmittel bei einer Temperatur zwischen 25 und 45°C durchführt und der Ester in Form des Natriumsalzes anschließend durch Ausfällung mithilfe von 10 Gew.-% Natriumacetat in einem Alkohol bei einem Verhältnis von 1 bis 1,2 Volumina Alkohol pro Volumen des Reaktionsmediums gewonnen wird.

16. Verfahren nach Anspruch 13 oder 15, **dadurch gekennzeichnet, dass** der Veresterungsgrad des quaternären Ammoniumsalzes des Benzylesters von Heparin zwischen 50 und 100% beträgt.

17. Verfahren nach Anspruch 13, 15 oder 16, **dadurch gekennzeichnet, dass** man 0,5 bis 1,5 Gewichtsteile Benzylchlorid auf 1 Gewichtsteil des Benzethoniumsalzes von Heparin verwendet, wobei die Reaktionsdauer zwischen 10 und 35 Std beträgt.

18. Verfahren nach Anspruch 13, **dadurch gekennzeichnet, dass** man die Umsalzung von Schritt c) mithilfe eines quaternären Ammoniumchlorids in wässrigem Medium bei einer Temperatur zwischen 10 und 25°C durchführt.

19. Verfahren nach Anspruch 18, **dadurch gekennzeichnet, dass** das Molverhältnis von quaternärem Ammoniumchlorid/Natriumsalz des Benzylesters von Heparin zwischen 2 und 3 beträgt.

20. Verfahren nach Anspruch 13, **dadurch gekennzeichnet, dass** man die Umwandlung des quaternären Ammoniumsalzes des Benzylesters des depolymerisierten Heparins in das Natriumsalz (Schritt e) durch Behandlung des Reaktionsmediums mit einer alkoholischen Natriumacetatlösung bei einer Temperatur zwischen 15 und 25°C durchführt.

21. Verfahren nach Anspruch 13, **dadurch gekennzeichnet, dass** die Verseifung (Schritt f) mithilfe eines Alkalimetallhydroxids in wässrigem Medium bei einer Temperatur zwischen 0 und 20°C durchgeführt wird.

22. Verfahren nach Anspruch 21, **dadurch gekennzeichnet, dass** man 1 bis 5 Moläquivalente Alkalimetallhydroxid verwendet.

23. Verfahren nach Anspruch 13, **dadurch gekennzeichnet, dass** man mithilfe von Wasserstoffperoxid in wässrigem Medium bei einer Temperatur von 10 bis 50°C reinigt (Schritt g).

24. Arzneimittel, das die Gemische von Oligosacchariden nach einem der Ansprüche 1 bis 6 enthält.

25. Arzneimittel nach Anspruch 24, **dadurch gekennzeichnet, dass** es eine antithrombotische Aktivität aufweist.

26. Arzneimittel nach den Ansprüchen 24 oder 25 zur Vorbeugung oder Behandlung von venösen und arteriellen Thrombosen, tiefer Venenthrombose, Lungenembolie, instabiler Angina pectoris, Myokardinfarkt, Herzischämie, Verschlusskrankheiten der peripheren Arterien, Vorhofflimmern, der Proliferation glatter Muskelzellen, von Atherosklerose und Arteriosklerose, Krebs durch Modulation der Angiogenese und der Wachstumsfaktoren sowie von diabetischen Störungen.

27. Pharmazeutische Zusammensetzungen, die mindestens ein Arzneimittel nach Anspruch 24 und einen oder mehrere pharmazeutisch inerte Excipienten oder Vehikel oder Zusatzstoffe enthält.

28. Pharmazeutische Zusammensetzungen nach Anspruch 27, **dadurch gekennzeichnet, dass** sie aus Lösungen bestehen, die auf subkutanem oder intravenösem Weg injizierbar sind.

29. Pharmazeutische Zusammensetzungen nach Anspruch 27, **dadurch gekennzeichnet, dass** sie aus einer Formulierung zur Inhalation über den pulmonalen Verabreichungsweg bestehen.

30. Pharmazeutische Zusammensetzungen nach Anspruch 27, **dadurch gekennzeichnet, dass** sie aus einer Formulierung für die orale Verabreichung bestehen.

31. Gemische von sulfatisierten Oligosacchariden nach Anspruch 1, die durch das Verfahren nach Anspruch 13 erhalten werden können.
